# EUROPEAN PATENT SPECIFICATION

(11) **EP 1 948 315 B1**
(45) Date of publication and mention of the grant of the patent: **12.08.2009**
(21) Application number: 06839751.2
(22) Date of filing: 07.11.2006
(51) Int. Cl.: A61N 7/00, G10K 11/00, A61B 19/00

(54) **APPARATUS FOR MOUNTING AN ULTRASONIC THERAPEUTIC DEVICE TO AN ORTHOPAEDIC CAST**
VORRICHTUNG ZUR MONTAGE EINES ULTRASCHALLTHERAPIEGERÄTES AN EINEM ORTHOPÄDISCHEN GIPS
APPAREIL POUR MONTER UN DISPOSITIF THERAPEUTIQUE A ULTRASONS SUR UN MOULAGE ORTHOPEDIQUE

(30) Priority: 07.11.2005 US 734245 P
(43) Date of publication of application: 30.07.2008
(73) Proprietor: Smith and Nephew, Inc., Memphis, TN 38116 (US)
(72) Inventor: TANIS, Kevin, J., Collierville, TN 38017 (US); ARRINGTON, Debra Ann, Cordova, TN 38018 (US); SCHNEIDER, John B., Memphis, TN 38117 (US)
(74) Representative: Adcocks, Clair
(86) International application number: PCT/US2006/060628
(87) International publication number: WO 2007/056734

(56) References cited:
- WO-A-96/25112
- WO-A-99/47208
- US-A- 5 186 162

## Description

### Background of the Invention

### 1. Field of the Invention

This invention relates generally to therapeutic ultrasound devices and, more particularly, to an apparatus and method for mounting a therapeutic device to an orthopaedic cast or other medical wrapping.

### 2. Related Art

The use of ultrasound to therapeutically treat and evaluate bone injuries is known. Impinging ultrasonic pulses having appropriate parameters, e.g., frequency, pulse repetition, and amplitude, for suitable periods of time and at a proper external location adjacent to a bone injury has been determined to accelerate the natural healing of, for example, bone breaks and fractures. For patients with reduced healing capacity, such as elderly persons with osteoporosis, ultrasonic therapy may promote healing of bone injuries that would otherwise require prosthetic replacement or leave the patient permanently disabled.

The ultrasound therapy is often used in conjunction with medical wraps, such as an orthopaedic cast. A rigid or semi-rigid plastic transducer port is mounted onto a fracture cast allowing its use for all large and small bones. Providing reliable bonding of the port into the cast with minimal increase in elevation and radius is highly desirable. Currently, reliance is placed on the adhesive properties of the cast resin in shear to bond the port to the cast. This is a disadvantage as a shear bond is not as strong as other types of bonding.

Another problem associated with the prior art transducer mounting apparatus becomes apparent to physicians during the installation of the apparatus. Typically, a cast will be mounted on the patient prior to the time that the decision is made to administer ultrasound therapy. Therefore, the physician is required to cut a hole in the existing cast to accommodate placement of an ultrasound transducer head module adjacent a body portion of a patient requiring treatment. Because a substantial number of transducer head modules are circular, a corresponding circular hole is required in the cast. However, physicians are commonly equipped with a tool having a blade that may be adjusted to limit penetration to the depth of the cast to cut a square or rectangular void in the cast. Moreover, it is inefficient to require the physician to be concerned with the precision with which the void is made in the cast. Therefore, a need exists for an apparatus that can be placed within a void in a cast and convert the square or rectangular void to a circular hole for receiving an ultrasound transducer head module and also an apparatus that is adaptable and versatile to minimize a precision associated with the dimensions of the void.

Typically, an ultrasonic therapy device may be applied to a cast in one of three ways. First, a medical practitioner may cut a hole in the cast and strap an ultrasonic transducer directly over the hole. Second, the medical practitioner may cut a hole in the cast and force fit a plastic transducer port into the cast, and then place the ultrasonic transducer in the port. Third, the medical practitioner may build a cast around a plastic transducer port and thereafter mount the ultrasonic transducer in the port.

Alternatively, the physician may know, at the time the injury occurs, that ultrasound therapy is likely a preferred future treatment. However, the installation of a spacer which creates a void in the cast has heretofor been delayed until a period of time has elapsed such that the danger of swelling around the affected injury site has transpired, because it has been determined that the skin within the void is prone to window edema (especially during the swelling period). Therefore, a need exists for an apparatus that allows the surgeon to install an insert or support fixture in the cast at the time of injury which will insertably receive an ultrasound transducer treatment head module and also prevent window edema when the module is not in place.

There remains a need in the art for an improved assembly for accurately mounting and positioning a therapeutic treatment device onto a cast that overcomes the above-noted disadvantages, is easy to use, and provides better results in healing musculoskeletal and bone injuries.

The publication WO 99/47208, which is considered as closes prior art document for the invention, discloses an apparatus for ultrasonically treating an injury in conjunction with an orthopaedic cast, the apparatus comprising a portable self-contained main operating unit, a support fixture configured and adapted for attachment to the orthopaedic cast adjacent an external site corresponding to an internal injury remote from said main operating unit, said support fixture having a body and a padding projection extending from said body, an ultrasonic transducer treatment head module operatively connected to said main operating unit and detachably engaged with said body of said support fixture, and casting material for attaching the support fixture to the orthopaedic cast,
wherein at least a portion of the casting material impregnates the padding projection. In this publication, the structure of the padding forming the padding projection is not specified.

### Summary of the Invention

It is in view of the above problems that the present invention was developed. According to an aspect of the invention there is provided an apparatus for ultrasonically treating an injury in conjunction with an orthopaedic cast. The apparatus includes: a portable self-contained main operating unit; a support fixture configured and adapted for attachment to the orthopaedic cast adjacent an external site corresponding to an internal injury remote from the main operating unit, the support fixture having a body and at least one mesh projection extending from the body; an ultrasonic transducer treatment head module operatively connected to the main operating unit and detachably engaged with the body of the support fixture; and casting material for attaching the support fixture to the orthopaedic cast, wherein at least a portion of the casting material impregnates the at least one mesh projection.

In one embodiment of the invention, the at least one mesh projection comprises a planar mesh base.

In another embodiment of the invention, the at least one mesh projection comprises a plurality of mesh tabs.

In another embodiment of the invention, the at least one mesh projection further comprises at least one living hinge.

In yet another embodiment of the invention, the at least one mesh projection conforms to a small radius of curvature.

In one particular embodiment of the invention, the main operating unit has an internal power source and is dimensioned to be carried by a patient during treatment.

In another embodiment of the invention, the treatment head module has an ultrasonic signal generator and signal generator circuitry operatively associated therewith.

In yet another embodiment of the invention, the treatment head module comprises an ultrasound transducer.

In still another embodiment of the invention, the ultrasound transducer has piezoelectric properties and is made from a material selected from the group consisting of a ceramic material, a single-crystal relaxor ferroelectric, lead zirconate titanate, lead metaniobate, barium titanate, and piezoelectric co-polymers of polyvinylidene fluoride (PVDF).

In another embodiment of the invention, the treatment head module is connected to the main operating unit through a wireless connection.

In one particular embodiment of the invention, the main operating unit has an ultrasonic signal generator and signal generator circuitry, wherein the signal generator circuitry includes a processor, a pulsed signal generator, and a switch coupled to the processor for regulating the pulsed signal.

In another embodiment of the invention, the main operating unit has a display panel coupled to the signal generator circuitry to display treatment sequence data and a keypad coupled to the signal generator circuitry to permit user control of the signal generator.

In another embodiment of the invention, there is provided an optical transmitter connected to the switch, the optical transmitter being configured to convert the pulsed signal to an optical signal.

In yet another embodiment of the invention, there is provided a communication interface connected between a communication port and the processor to provide a communication link between the ultrasonic signal generator and an external computer/modem..

In still another embodiment of the invention, there is provided an alarm connected to the processor to indicate accurate compliance with a treatment protocol.

In one particular embodiment of the invention, the body of the support fixture has an aperture configured to receive a portion of the ultrasonic transducer treatment head module.

In another embodiment of the invention, the body has at least two bayonet lugs extending into the aperture which are electrically connected to form a conductive path therebetween.

In yet another embodiment of the invention, the ultrasonic transducer treatment head module includes at least two slotted lugs having at least a portion thereof extending from an outer surface of the module and configured to engage the at least two bayonet lugs, the at least two slotted lugs being fabricated from conductive plastic such that when the slotted lugs engage the bayonet lugs a conductive path is formed between the slotted lugs.

In another embodiment of the invention, the support fixture has an outer surface and an inner surface defining an axial bore therethrough with a proximal inlet and a distal outlet.

In yet another embodiment of the invention, there is provided a spacer configured to fit within a void in the orthopaedic cast, the spacer having an opening therein, the opening having a shape corresponding to an outer periphery of the support fixture, and the support fixture being at least partially positioned within the opening. In one particular embodiment of the invention, the spacer is formed of felt.

In another embodiment of the invention, there is provided an ultrasound transmission-enhancing medium positioned within the body. In one particular embodiment of the invention; the ultrasound transmission-enhancing medium is a gel pad.

In another embodiment of the invention, there is provided a locking structure on an outside periphery of the body.

In still another embodiment of the invention, the body extends upwardly in a transverse direction relative to the at least one mesh projection.

In yet another embodiment of the invention, there is provided a cap attached to the body and a biasing element connected to the cap, wherein the biasing element engages the ultrasonic transducer treatment head module.

In another embodiment of the invention, the body is a cylindrical hollow tube.

In yet another embodiment of the invention, the body further comprises a lip.

In still another embodiment of the invention, the body has a proximal end portion and a distal end portion, and the at least one mesh projection is located at the distal end portion.

In another embodiment of the invention, the casting material comprises at least one cast material strip.

In yet another embodiment of the invention, the support structure further comprises a hemispherical notch.

In another embodiment of the invention, the mesh projection is made from a material selected from the group consisting of a polymer or a composite. In, one particular embodiment of the invention, the polymer is selected from the group consisting of thermoplastic polymers, thermosetting polymers, and elastomers. In another embodiment of the invention, the polymer is made of a fourteen count polyester core yarn having a vinyl coating.

In one particular embodiment of the invention, the mesh projection is made from a material selected from the group consisting of polyvinyl chloride, polyethylene, acrylonitrile butadiene styrene, or silicone.

In another embodiment of the invention, the mesh projection has a thickness A, and the thickness A is in the range from about one-half millimeter to about seven millimeters. In one particular embodiment of the invention, the thickness A is about one millimeter.

In another embodiment of the invention, the mesh projection has a first dimension D1 and a second dimension D2, and the first dimension D1 is in the range from about twenty-five millimeters to about one hundred fifty millimeters, and the second dimension D2 is in the range from about twenty-five millimeters to about one hundred fifty millimeters. In one particular embodiment of the invention,D1 and D2 are each about 57 millimeters.

In another embodiment of the invention, the body is rigid or semi-rigid.

In another embodiment of the invention, a portion of the mesh projection is constructed of a material that may be seen with a chosen medical visualizing system.

In another embodiment of the invention, a portion of the mesh projection is at least partially opaque to X-radiation..

In another embodiment of the invention, a portion of the mesh projection is at least partially opaque to infra-red radiation.

In another embodiment of the invention, a portion of the mesh projection is at least partially paramagnetic.

In another embodiment of the invention, a portion of the mesh projection includes an adhesive coating.

In another embodiment of the invention, the mesh projection further comprises one or more peripheral markers. In one particular embodiment of the invention, there are four peripheral markers. In another embodiment of the invention, the peripheral markers are selected from the group consisting of a radio-opaque thread woven into the mesh, radio-opaque ink, cut pieces of lead tape.

In another embodiment of the invention, there is provided a central marker.

In another embodiment of the invention, the mesh projection has a varying weave spacing.

In another embodiment of the invention, the support fixture has a concave lower end.

In another embodiment of the invention, the support fixture includes at least one circumferential groove in an upper portion thereof.

In another embodiment of the invention, the support fixture includes at least one circumferential flange.

In another embodiment of the invention, the support fixture features locking structure on the outside periphery of the body.

In another embodiment of the invention, the locking structure includes a circumferential ridge on the outside periphery of the support fixture which is configured to engage at least one locking member extending downward from an outer periphery of a cover. In one particular embodiment of the invention, there are three locking members.

In another embodiment of the invention, the locking member is formed of a resilient material such that it will flex outward as the ridge thereon is forced over ridge, and it will snap back into position after it moves beyond ridge.

In another embodiment of the invention, there is provided a support fixture configured and adapted for attachment to a medical wrap adjacent an external site corresponding to an internal injury, the support fixture comprising: a body, the body having an inner wall and an outer wall, the inner wall forming an opening adapted to receive, in use, an ultrasonic therapy device; and at least one mesh projection, the at least one mesh projection extending from the body and having a plurality of openings, and wherein at least a portion of the plurality of openings is impregnated with a portion of the medical wrap.

The invention has several advantages over prior devices and techniques. First, the apparatus has increased strength in comparison to existing ports due to the impregnation of the mesh projection by the casting material, such as resin. Second, the mesh projection provides the ability to use the fixture on smaller radius limbs. Third, the mesh projection allows for a reduction in size of the cast opening, thereby reducing the possibility of cast failure.

Thus, in furtherance of the above goals and advantages, the present invention is, briefly, a rigid or semi-rigid transducer body with a flexible mesh or weave projection which enables conformation to a small radius of curvature, for example, around a limb or finger. The weave is sufficiently open to allow resin impregnation through the weave for incorporation of the transducer body to the cast. The resin impregnated weave does not rely on shear properties (which are weaker) for attachment to the cast but instead relies on the strength of the resin in tension and compression as the resin passes through the weave and attaches to the cast.

Further features, aspects, and advantages of the present invention, as well as the structure and operation of various embodiments of the present invention, are described in detail below with reference to the accompanying drawings.

### Brief Description of the Drawing

The accompanying drawings, which are incorporated in and form a part of the specification, illustrate embodiments or the present invention and together with the description, serve to explain the principles of the invention. In the drawings:

Figure 1 is a perspective view of a portable ultrasonic treatment apparatus according to the present invention, illustrating a main operating unit or controller and an ultrasonic transducer treatment head module;

Figure 2 is an exploded view of the main operating unit;

Figure 3 is a block diagram of the circuitry for the main operating unit;

Figure 4 is a block diagram of one embodiment of the circuitry for the ultrasonic transducer assembly;

Figure 5 is a block diagram of an alternative embodiment of the circuitry for the ultrasonic transducer assembly;

Figure 6 is a schematic diagram illustrating an alternative embodiment of the portable ultrasonic treatment apparatus;

Figure 7 is a sectional side view of an ultrasonic transducer assembly to deliver ultrasound waves into a medium B;

Figure 8 is a top view of a fixture with a cap mounted thereon;

Figure 9 is a top view of the fixture alone;

Figure 10 is a perspective view of a locating ring and strap for locating bone injuries;

Figure 11 is a perspective view of the locating ring of Figure 10 affixed to a patient wearing a cast and illustrating a mark to define the location of a bone injury;

Figure 12 is a top view of a mesh base in a second embodiment;

Figure 13 is a perspective view of a template centrally located over the mark;

Figure 14 is a perspective view with parts separated of the patient's cast with a removed section and a fixture for retaining and aligning the ultrasonic transducer assembly of Figure 1;

Figure 15 is a perspective top view of the fixture located on a cast;

Figure 16 is a perspective view of the fixture being secured to the cast at the removed section;

Figure 17 is a perspective view with parts separated of the cast, the fixture and a cap for the fixture;

Figure 18 is a top view of a template in a second embodiment;

Figure 19 is a side perspective view of the mesh base being used as a template;

Figure 20 is a perspective view with parts separated, illustrating the ultrasonic transducer assembly aligned for releasable attachment to the fixture;

Figure 21 is a top view of the fixture within a void in a cast;

Figure 22 is a side cross-sectional view of a fixture partially secured within a void in a cast;

Figure 23 is a top view of the fixture having a plurality of tabs extending radially therefrom;

Figure 24 is an exploded side view of an apparatus for mounting an ultrasound transducer;

Figure 25 is an enlarged side view of an assembled apparatus for mounting an ultrasound transducer;

Figure 26 is a perspective view of the fixture secured in a cast ready to receive an ultrasound transducer head;

Figure 27 is a perspective view of a fully assembled apparatus for mounting an ultrasound transducer in a cast;

Figure 28 is a partial enlarged side view of another embodiment of an apparatus for mounting an ultrasound transducer;

Figures 29-32 are various views of a cover illustrating alternative locking structure;

Figure 33 is an exploded side view of an apparatus for mounting an ultrasound transducer having a cover with external locking structure;

Figure 34 is a perspective view of another embodiment of a cover with alternative locking structure;

Figure 35 is a side view in cross-section of an apparatus for the installation of the fixture adjacent a treatment location prior to installing a cast thereon;

Figure 36 is a perspective view of a piece of casting tape and a sealed package therefor;

Figures 37-39 are perspective views illustrating a system for mounting an ultrasound transducer receiving apparatus adjacent a treatment location; and

Figures 40-42 are perspective views illustrating a system for mounting an ultrasound transducer receiving apparatus adjacent a treatment location in a cast.

### Detailed Description of the Embodiments

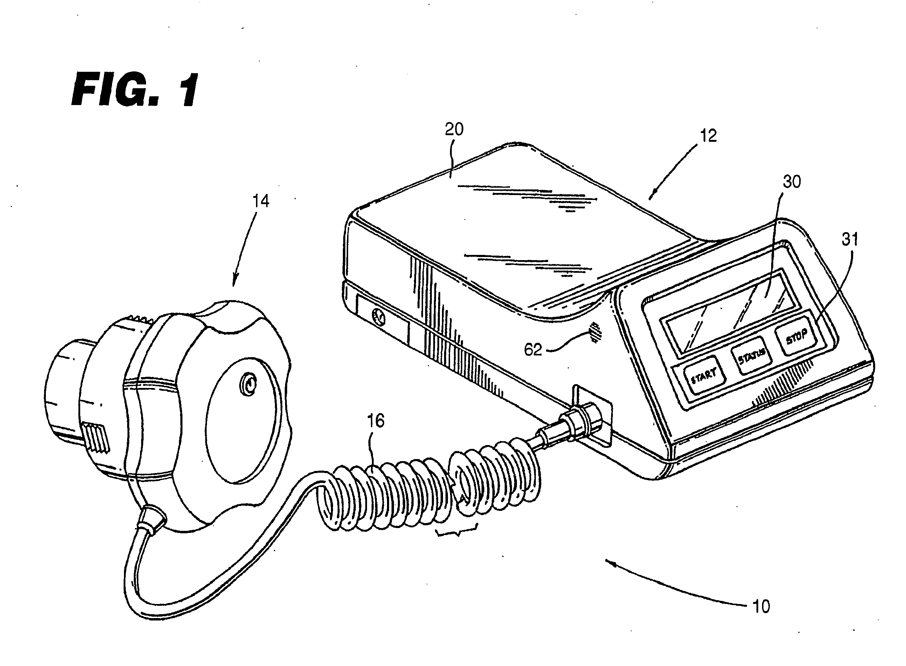

Referring to the accompanying drawings in which like reference numbers indicate like elements, Figure 1 illustrates a portable ultrasonic treatment apparatus 10. The ultrasonic treatment apparatus 10 includes a portable main operating unit (MOU) 12 and an ultrasonic transducer treatment head module 14 coupled to the MOU 12 by a first cable 16. The MOU 12 provides control signals for the ultrasonic transducer treatment head module 14. The first cable 16 may be a multi-conductor cable capable of transmitting relatively low frequency or optical signals, as well as digital signals. The first cable 16 may include coaxial cable or other type of suitable shielded cable. Alternatively, the first cable 16 may include fiber optic cable for transmitting optical signals. In operation, the transducer treatment head module is positioned adjacent the injured area and excited for a predetermined period of time. To ensure that the transducer treatment head module is properly positioned, a safety interlock may be provided to prevent inadvertent excitation of the transducer assembly and to insure patient compliance. Although shown herein with a single transducer treatment head module, the present invention also envisions a plurality of head modules for use with a single MOU.

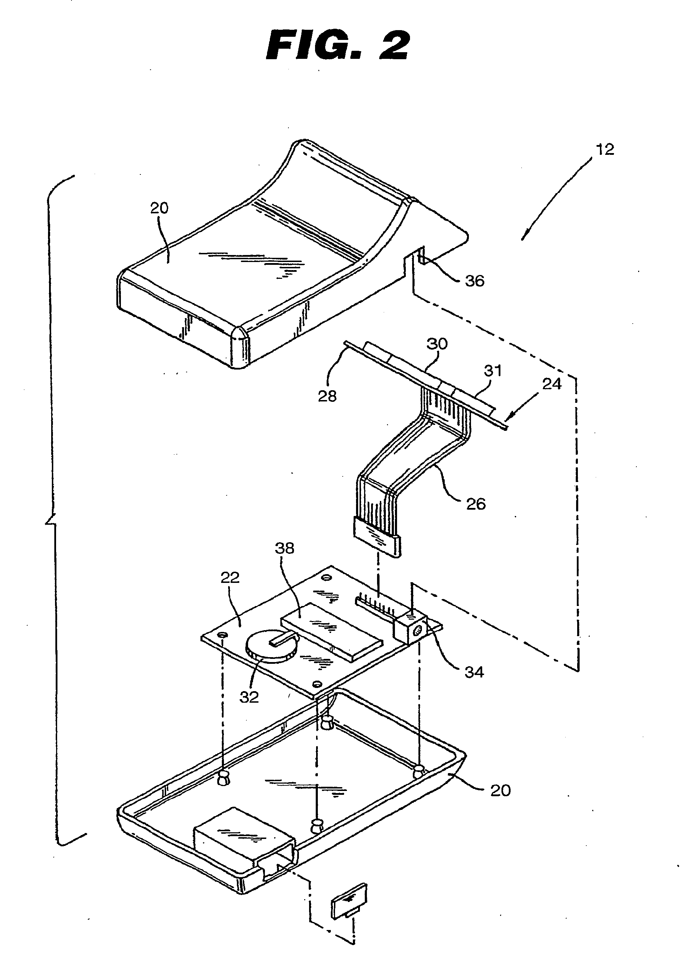

Referring to Figure 2, MOU 12 includes a housing 20 which is typically constructed in two half-sections joined together by screws, ultrasonic welds or adhesives. A printed circuit board 22 is positioned within the housing 20 and coupled to display assembly 24 via a second cable 26. Display assembly 24 includes mounting board 28, display 30 and a keypad 31, shown in Figure 1. Display 30 may be, for example, a liquid crystal type display or an LED type display suitable for displaying text and numerals. Battery holder 32 is connected to printed circuit board 22 for portable operation of the real time clock and the ultrasonic treatment head module of the present invention. In addition, a suitable battery, such as a bank of three (3) lithium batteries, is positioned in the battery compartment.

Communication port 34 is affixed to printed circuit board 22 and accessible through channel 36 in housing 20. Communication port 34 is coupled to signal generator circuitry 38 on printed circuit board 22 and provides a communication link, e.g., for serial communications, between the MOU 12 and an external computer. In this configuration, a physician can download information, such as the number, date, time of day, and/or duration of actual treatments initiated by the patient, stored within signal generator circuitry 38.

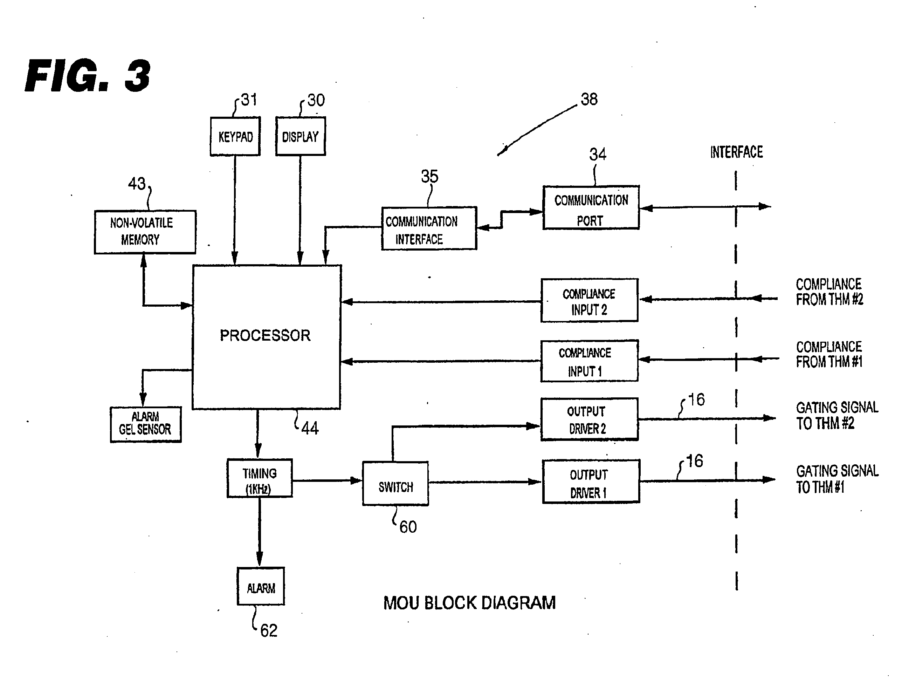

Figure 3 illustrates a block diagram of the signal generator circuitry 38 within the MOU 12 which generates and controls the pulses transferred to the ultrasonic transducer assembly 14. Signal generator circuitry 38 includes a processor 44 having memory 43 (e.g., RAM and ROM) and stored programs (e.g., system and application) for controlling the operation of the processor, as well as the transducer treatment head module 14. Processor 44 is coupled to display 30 and keypad 31 and is configured to receive data from the keypad 31 and to transfer data to the display 30. Processor 44 may include a microprocessor or processor 44 may be a microcontroller having internal memory. Communication interface 35 is connected between communication port 34 and processor 44 and is provided to communicate with, for example, an external computer. Communication interface 35 may be a serial interface, such as an RS-232 interface, a parallel interface, a universal serial bus interface, or a modem.

Processor 44 is also utilized to control the treatment sequence, i.e., the start time and the stop time of the ultrasonic treatment. The processor may be preprogrammed for treatment times and the user (e.g. the physician or patient) selects one of the treatment times via keypad 31, or the processor may be programmed by the user via keypad 31 to set the start and stop sequence. Treatment times may range between about one and about sixty minutes, although treatments in the order of 10-20 minutes are typical. When the treatment time is activated, processor 44 closes switch 60 which permits the modulated signal to pass to cable 16. When the treatment time expires, switch 60 is opened and the modulated signal is inhibited from passing to cable 16. In addition, when the treatment time expires, processor 44 may send an alarm signal to alarm 62 which activates.

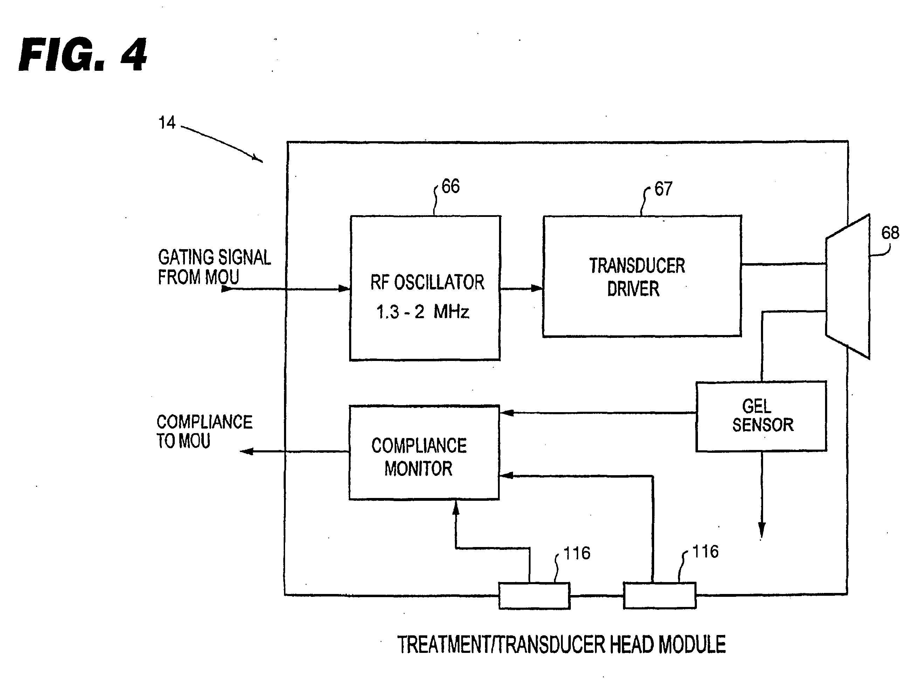

Referring to Figure 4, a block diagram of one embodiment of the transducer treatment head module circuitry is shown. The transducer treatment head module circuitry includes a receiver 66 which receives the signals transferred by MOU 12 via cable 16. Receiver 66 is connected to transducer driver 67 which excites transducer 68.

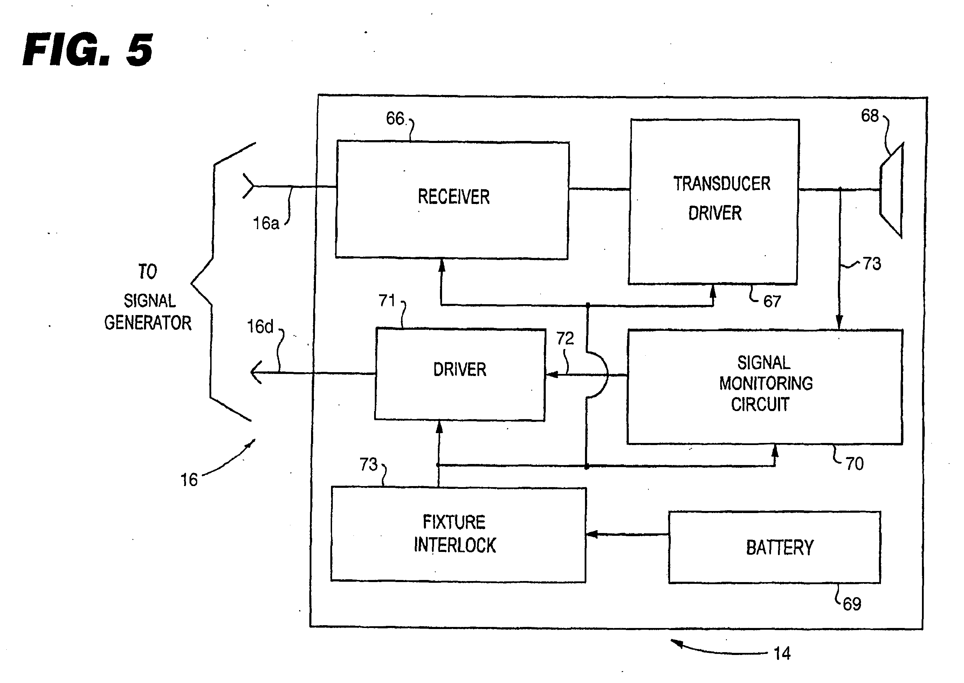

An alternative embodiment of the transducer treatment head module circuitry is shown in Figure 5. In this embodiment, the transducer treatment head module 14 includes an internal battery 69 which supplies power to the internal components of the transducer treatment head module. For example, battery 69 supplies power to signal monitoring circuit 70 and signal driver 71. The signal monitoring circuit 70 provides a digital output signal 72 which represents the waveform characteristics of the output of transducer driver 67. Such characteristics may include, for example, the frequency, pulse repetition frequency, the pulse width and the average output power of the signal driving transducer 68. The output signal 72 of signal monitoring circuit 70 is transferred to the MOU 12 via driver 71 and the first cable 16. Optional fixture interlock 73, which may include switches on the outer surface of the transducer treatment head module, provides power to the internal components of the transducer treatment head module 14 so as to ensure that the transducer treatment head module 14 is properly positioned before the transducer is excited.

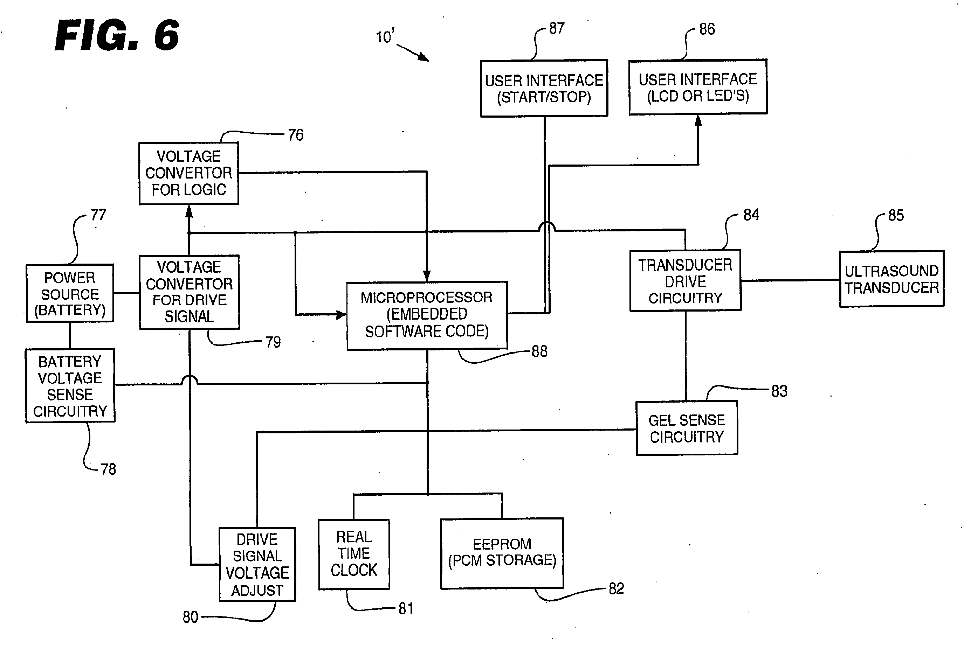

Figure 6 illustrates an alternative embodiment of the ultrasonic treatment apparatus, generally indicated by reference numeral 10'. The ultrasonic treatment apparatus 10' includes a logic voltage convertor 76, a power source 77, battery voltage sense circuitry 78, a drive signal voltage converter 79, a drive signal voltage adjustor 80, a real time clock 81, an EEPROM 82, gel sense circuitry 83, a transducer drive circuitry 84, an ultrasound transducer 85, a first user interface 86, a second user interface 87, and a microprocessor 88. The configuration of the ultrasonic treatment apparatus 10' allows for most of the components to be placed in the MOU 12 and only the ultrasound transducer 85 placed in the head module 14. In some embodiments, the head module 14 may consist solely of the ultrasound transducer 85.

The logic voltage converter 76 steps down the voltage for the microprocessor and other electronics. The power source 77 supplies power to the ultrasonic treatment apparatus 10' and may be a battery or battery pack. The battery voltage sense circuitry 78 senses the power of the power source 77 and prevents operation if there is insufficient power to drive the transducer and the other electronic components. The drive signal voltage converter 79 steps down the voltage for the transducer drive circuitry 84. The drive signal voltage adjustor 80 is used to calibrate the transducer drive circuitry 84 to obtain a predetermined acoustic wave from the transducer 85. The real time clock 81 is the clock for the microprocessor 88. The EEPROM 82 is used to record data after a treatment. Such data may include date, time, length of treatment, and other variables.

The gel sense circuitry 83 senses an impedance change through the transducer to ascertain whether the transducer is emitting acoustic waves into a fluid or gel medium. Every transducer has an impedance when it is acting on a medium, such as air or water. The impedance is different for each medium. By using this difference, the gel sense circuitry 83 can determine whether or not the transducer is acting upon a gel medium.

The transducer drive circuitry 84 creates the sinusoidal wave that drives the transducer 85. The ultrasound transducer 85 creates the ultrasonic wave for treating the injury. The first user interface 86 is a display. The first user interface 86 may indicate items such as time duration, and errors. The second user interface 87 is one or more switches that are used to control the ultrasonic treatment apparatus 10'. Such switches may include a start button, a stop button, and a reset button. The microprocessor 88 executes stored programs to apply the treatment in a predefined manner.

Further, some embodiments of the ultrasonic treatment apparatus 10' eliminate the wired connection between the transducer drive circuitry 84 and the ultrasound transducer 85 and instead implement a wireless connection, such as ZIGBEE^{™} , BLUETOOTH^{™}, IEEE 802.11, or other Radio Frequency (RF) technology. ZigBee is a published specification set of high level communication protocols designed for wireless personal area networks (WPANs). The ZIGBEE trademark is owned by ZigBee Alliance Corp., 2400 Camino Ramon, Suite 375, San Ramon, California, U.S.A. 94583. Bluetooth is a technical industry standard that facilitates short range communication between wireless devices. The BLUETOOTH trademark is owned by Bluetooth Sig, Inc., 500 108th Avenue NE, Suite 250, Bellevue Washington, U.S.A. 98004. IEEE 802.11 denotes a set of Wireless LAN/WLAN standards developed by working group 11 of the IEEE LAN/MAN Standards Committee (IEEE 802). RF is a wireless communication technology using electromagnetic waves to transmit and receive data using a signal above approximately 0.1 MHz in frequency.

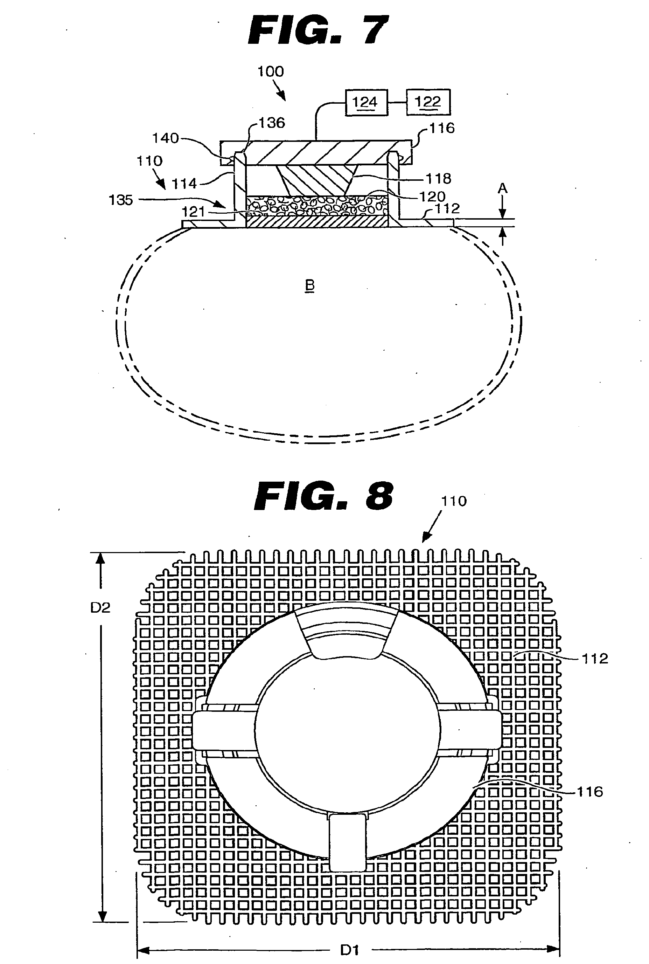

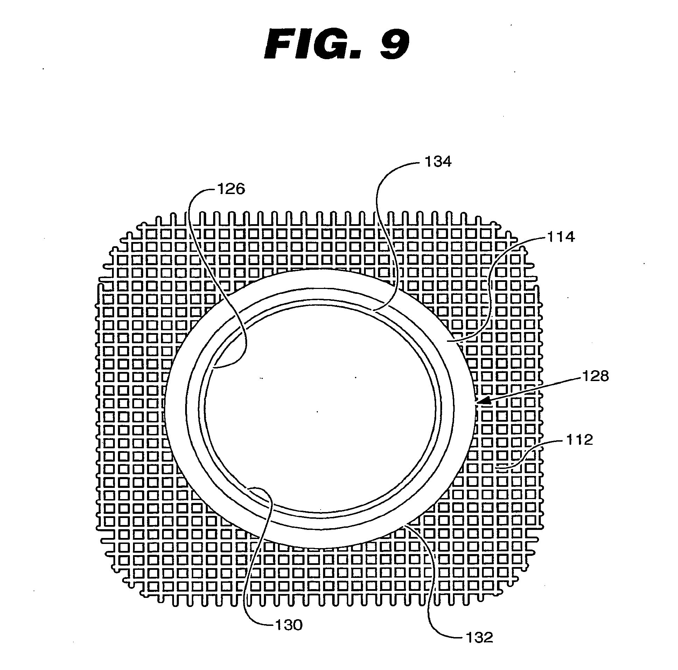

Figures 7-9 illustrate an ultrasonic transducer assembly 100 to deliver ultrasound waves into a medium B, which may be composed of soft tissue or bone in order to promote healing of a wound in the soft issue and/or healing of a fracture in a bone. The ultrasonic transducer assembly 100 includes a support fixture 110. The support fixture may also be termed a transducer port or simply a port. The support fixture 110 includes a body 114 and a mesh projection 112 that extends from the body 114. In the depicted embodiments, the mesh projection 112 is a planar mesh base, and the body 114 that extends upwardly from the mesh base 112 in a transverse direction. The ultrasonic transducer assembly 100 also includes a cap 116, a spring 118, and a transducer 120. In the depicted embodiments, the base 112 is shown assuming that the interface with the medium B is flat. However, those skilled in the art would understand that the base 112 can be shaped to fit the interface with medium B.

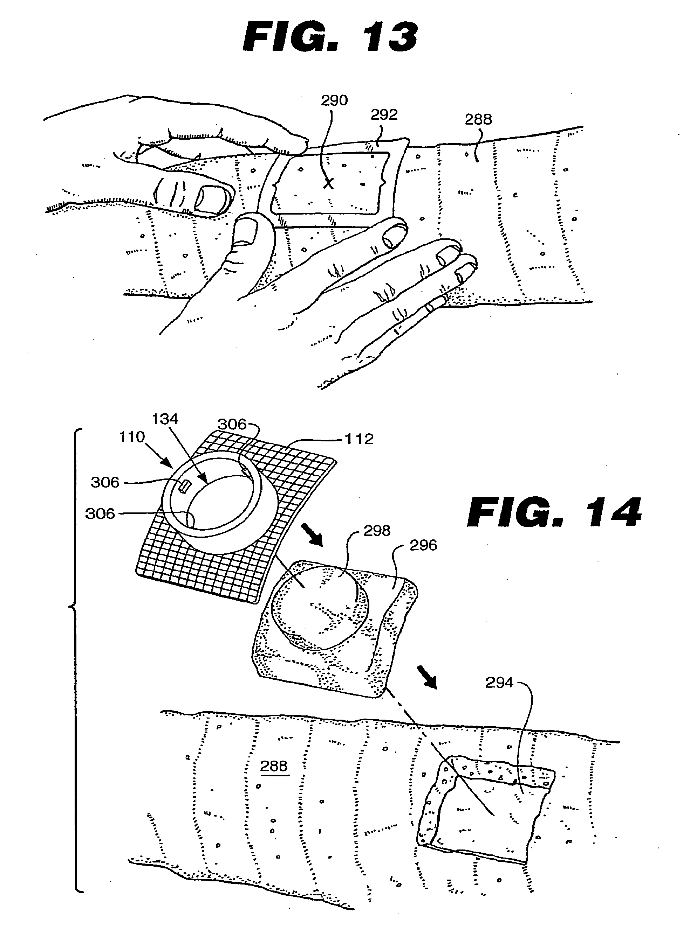

The base 112 is made of a mesh or woven material. The mesh or weave allows the base 112 to conform to a small radius of curvature. For example, the mesh or weave permits the base to conform to a small limb or finger. Further, the mesh or weave aids in the incorporation of the support fixture 110 to a cast 288 (best seen in Figure 13). As those skilled in the art understand, an orthopaedic cast is a shell that encases a limb (or, in some cases, large portions of the body) to hold a broken bone (or bones) in place until the broken bone has healed. Typically, the cast is formed of plaster, cotton bandages that have been impregnated with plaster of paris, or knitted fibreglass bandages impregnated with polyurethane. However, other materials may be used. As used herein, the term "resin" means a material that hardens to form a portion of the cast shell. While the depicted embodiments illustrate an orthopaedic cast, those having ordinary skill in the art would understand that other types of medical wrappings may be used:

As explained in greater detail below, the mesh or weave captures a portion of the cast resin, which strengthens the attachment of the support structure to the cast in comparison to prior devices. Prior devices utilized shear properties to retain the support structure to the cast. Impregnating the mesh or weave with resin permits the present invention to utilize shear, tension, and compression characteristics to adhere the support structure to the cast. This is a significant improvement over the state of the art.

The base 112 may be made from many different types of materials, such as a polymer or a composite. Polymers may include thermoplastic polymers, thermosetting polymers, and elastomers. Examples of polymers may include, among others, polyvinyl chloride, polyethylene, acrylonitrile butadiene styrene, or silicone. In the embodiment depicted in Figures 7-9, the base 112 is made of a fourteen count polyester core yarn having a vinyl coating. In the depicted embodiments, the base 112 has a square or rectangular shape, but those of ordinary skill in the art would understand that other shapes may be used. For example, the base 112 may be in the shape of a circle or triangle. The base 112 has a thickness A, which is in the range from about one-half millimeter to about seven millimeters. In the embodiment depicted in Figure 7, A is about one millimeter. The embodiment depicted in Figure 8 illustrates a first dimension D1 and a second dimension D2. The first dimension D1 is in the range from about twenty-five millimeters to about one hundred fifty millimeters. The second dimension D2 is in the range from about twenty-five millimeters to about one hundred fifty millimeters. In the embodiment depicted in Figures 6-8, D and D2 are each about 57 millimeters. The mesh base 112 may be oversized such that it can be cut-to-fit for a custom application of the support structure 110.

The body 114 is rigid or semi-rigid. The body 114 is shaped to receive the transducer 120. The transducer 120 is constructed of materials and designs that are commonly used in ultrasound applications. The transducer 120 may have piezoelectric properties and may be made from, as examples, a ceramic material, a single-crystal relaxor ferroelectric, lead zirconate titanate, lead metaniobate, barium titanate, and piezoelectric co-polymers of polyvinylidene fluoride (PVDF). Alternatively, the transducer 120 may have magnetostrictive properties.

In the embodiment depicted in Figures 7-9, the body 114 is a cylindrical, hollow tube as the transducer 120 has a circular cross-section but other shapes may be used. The body 114 has an inner portion 126 and an outer portion 128. In the embodiments depicted in Figures 7-9, the body 114 has an inner wall 130 and an outer wall 132 which define the inner portion 126 and the outer portion 128. The inner wall 130 forms an aperture 134 that receives the transducer 120. The inner wall 130 of the body 114 is dimensioned such that the transducer 120 fits tightly within the body 114. The inner wall 130 may have a diameter the same as or slightly larger than the diameter of the transducer 120. For example, the diameter of the inner wall 130 may be about zero to about two mm larger than the diameter of the transducer 120. The body 114 also has a proximal end portion 135 and a distal end portion 136. In the depicted embodiment, the mesh base 112 is connected to the body 114 at the proximal end portion 135. However, the mesh base 112 may be at the very end of the proximal end portion 135 or slightly above it such that the mesh base 112 attaches a few millimeters above the proximal end portion 135.

A cap 116 is connected to the body 114 at the distal end portion 136. For example, the body 114 may have a lip 140 (best seen in Figure 7) such that the cap 116 snaps onto the body 114. Further, the spring 118 is mounted between the cap 116 and the transducer 120 in order to exert pressure on the transducer 120. In some embodiments, the spring 118 may be mounted to the cap 116. The spring 118 biases the transducer 120 toward the proximal end portion 135 of the body 114. In some embodiments, the ultrasonic transducer assembly 100 includes gel or a gel bladder 121 to aid in the transference of acoustic waves from the transducer 120 to the medium B.

A system controller 122 spatially and temporally controls the acoustic waves that emanate from the transducer 120. The design and fabrication of the system controller 122 are well known to those who practice the art. The system controller 122 is electrically connected to a signal generator 124, and the signal generator 124 is electrically connected to the transducer 120. The system controller 122 triggers the programmable signal generator 124 to produce ultrasonic excitation signals that are sent to the transducer 120. The transducer 120 receives the excitation signal and emits an acoustic longitudinal wave that propagates on to medium B. The system controller 122 and the signal generator 124 may take the form of the MOU 12 described above.

The transducer 120 produces specific sequential or simultaneous transmissions of acoustic waves, which is controlled by the system controller 122, in order to non-invasively irradiate or interrogate the medium B ultrasonically. The system controller 122 may be a programmable microprocessor, but may also include, though is not limited to, integrated circuits, analog devices, programmable logic devices, personal computers or servers. The timing sequences may be established by the user at any time or established during the manufacturing process.

The ultrasonic transducer assembly 100 may be used to administer therapeutic treatment composed of an ultrasound dosage administered once or twice a day, and repeated daily for several months to effectively stimulate the healing process. In some embodiments, one dosage of acoustic waves ranges between one and sixty minutes in length for the transducer 120. The ultrasonic transducer assembly 110 may be used to facilitate and enhance application of therapeutic ultrasound dosages to shallow or deep anatomical structures, or both, in an effort to expedite tissue wound healing, including both the endosteal and periosteal healing phases in the bone fracture healing process.

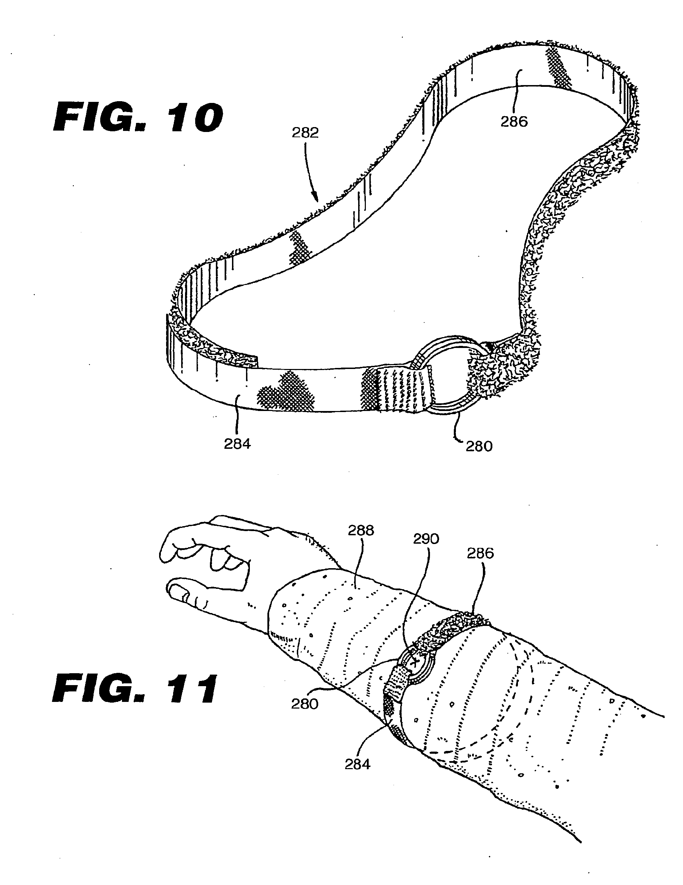

Referring now to Figure 10, a locating ring 280 for determining the location of injured bone is shown. The locating ring 280 includes a strap 282 for releasably securing the ring to a patient. The strap 282 preferably has two sections 284 and 286 which permit quick fastening and unfastening of the ring 280 on and off the patient. The ring 280 is constructed of material that may be seen with a chosen medical visualizing system. Thus, if X-rays are used, the ring 280 is at least partially opaque to X-radiation. If infra-red radiation is used, the ring 280 is at least partially opaque to infra-red radiation, and, if magnetic resonance imaging is used, the ring 280 is at least partially paramagnetic. However, the other materials may be used for the ring which permits detection by medical visualizing or imaging systems.

The dimensions of the ring 280 are typically a function of the size of the patient, the estimated size and location of the injury, and the type of visualizing system used. For example, to determine the location of a bone fracture in an average human limb, e.g., the ulna or radius, and using an X-ray imaging system, the diameter of the ring may nominally be about thirty-eight millimeters. In this example, the ring may be a rigid torus of metallic material, of cross-sectional diameter nominally five millimeters. As another example, if the visualization system utilized is an ultrasonic imaging system, the ring 280 may be substantially flexible and planar, so that it may contour to a surface it is placed adjacent to, thereby allowing the scanning or imaging transducer to be moved across the surface and the ring.

As noted, the strap 282 has two sections 284 and 286, each section has one end fastened to the ring 280. The two sections 284 and 286 may have hook and loop type fastening assembly, such as VELCRO^{™}, so that they may be fastened together and quickly unfastened. Other quick release fastening techniques are also contemplated.

Alternatively, a portion of the mesh base 112 may be used to identify the location of injured bone. Thus, a portion of the mesh base 112 may be constructed of a material that may be seen with a chosen medical visualizing system. For example, if X-rays are used, a portion of the mesh base 112 is at least partially opaque to X-radiation. If infra-red radiation is used, a portion of the mesh base 112 is at least partially opaque to infra-red radiation, and, if magnetic resonance imaging is used, a portion of the mesh base 112 is at least partially paramagnetic. However, the other materials may be used for the mesh base 112 which permits detection by medical visualizing or imaging systems. Additionally, the mesh base 112 may have an adhesive coating on one side for temporarily locating the mesh base 112 while the medical visualizing system is activated.

Figures 11 through 16 illustrate locating an injured bone, affixing the support fixture 110 configured to maintain the transducer treatment head module 14 adjacent the area of the injured bone, and connecting the ultrasonic transducer assembly 10 to the fixture 110 for treating the injured bone. Initially, the locator ring 280 is positioned on the cast 288 on, for example, a patient's arm, at a location corresponding to the estimated or approximated location of the injury. This initial position is a preliminary approximation of the external location of the bone injury, and may be based on previously taken X-rays, a physician's diagnosis or the patient's recall of the point of injury.

An external image, e.g., an X-ray, of the fractured region is taken to include the locating ring 280. Although the initial position of the locating ring 280 with respect to the bone injury is a preliminary approximation, in many instances the initial placement will be sufficiently accurate so that the X-ray will depict the bone injury framed by the ring 280. The resulting X-ray image indicates the position of the bone injury relative to the locating ring 280. The X-rays are used as a guide to locate and mark 290 the corresponding point on the cast relative to the actual locating ring 280. The mark 290 gives an approximate external location on the cast of the bone injury. If greater accuracy is required, the ring 280 may be centered about the mark 290, another X-ray is taken, and a new mark (not shown) is made on the cast based on the location of the bone fracture relative to the ring on the X-ray. Successive iterations of repositioning the locating ring 280 and X-raying the site will yield even greater accuracy.

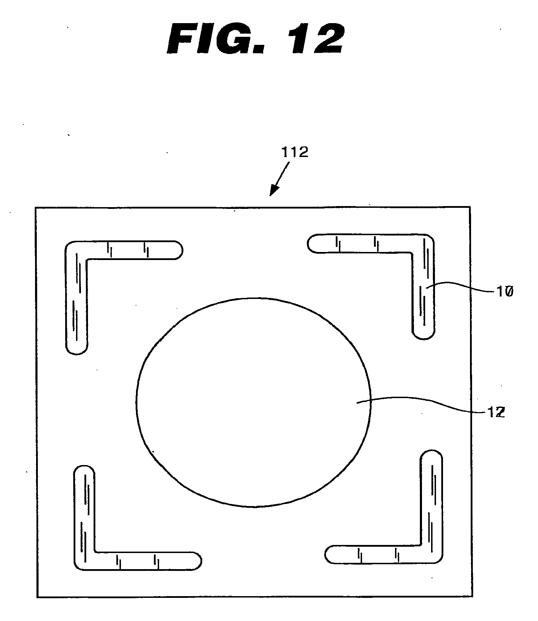

As noted above, in some embodiments, the mesh base 112 may be used instead of the ring 280 in order to locate the mark 290. Figure 12 illustrates one example of the mesh base 112 with one or more peripheral markers 170 that may be used in conjunction with a medical visualizing system, such as an x-ray machine. In the embodiment depicted in Figure 12, the mesh configuration of the mesh base 112 has been omitted for clarity. Additionally, in the embodiment depicted in Figure 12, there are four peripheral markers 170 but a greater or lesser number may be used. The peripheral markers 170 may be a radio-opaque thread woven into the mesh. Alternatively, the peripheral markers 170 may be radio-opaque ink. Finally, the peripheral markers 170 may be cut pieces of lead tape. Some embodiments may further include a central marker 172 to indicate the center of the mesh base 112. The peripheral markers 170 indicate the outline of the mesh base 112 while the central marker 172 may be used to locate the mark 290.

As shown in Figures 13 and 14, a first embodiment of a marking template 292 is pressed against the cast 288 and centered on the mark 290 of the external location on the cast 288 of the bone fracture. The outline of the inner edges of the rectangular template opening is traced on the cast 288, and the traced portion of the cast is removed so that the opening 294 in the cast 288 exposes the skin, as shown in Figure 14. The opening 294 in the cast 288 receives a felt pad 296 having a thickness approximately the same as the thickness of the cast. The felt pad 296 also has a cylindrical bore that receives a cylindrical felt pad 298. Felt pad 296 is provided to support the fixture 110 and to maintain pressure against the skin which helps prevent window edema (swelling) and is substantially equivalent to the pressure exerted by the cast 288 against the skin and is described in more detail below.

The template 292, and consequently the opening 294 in the cast 288, is smaller than the mesh base 112 of the fixture 110 for retaining and aligning the ultrasonic transducer assembly 14, so that the mesh base 112 engages the cast surface surrounding the opening 294 when the fixture 110 is placed over the opening 294. The fixture 110 includes the circular aperture 134 and may include bayonet locking lugs 306. Aperture 134 has substantially the same diameter as the cylindrical felt pad 298.

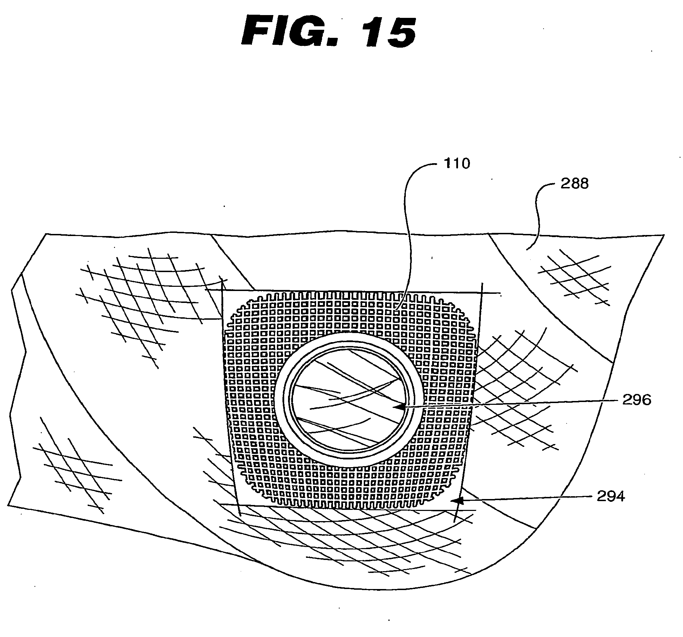

Figure 15 illustrates the fixture 110 placed over the felt 296 in the opening 294.

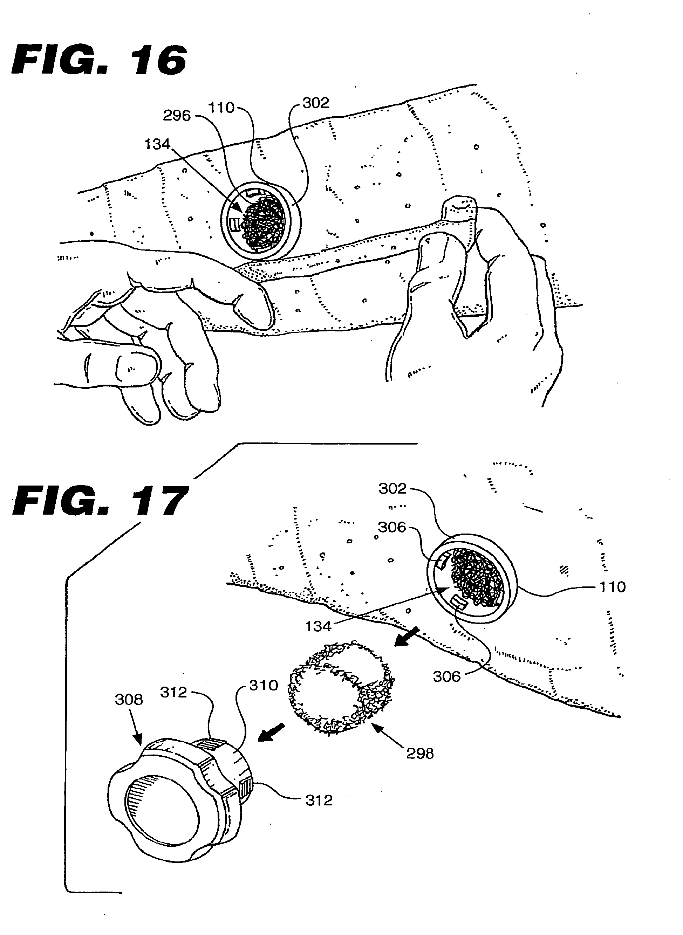

Figure 16 shows the fixture 110 positioned over the opening 294 in the cast 288 and the felt pad 296 so that the aperture 134 and the cylindrical felt pad 298 are coaxially aligned. The fixture 110 partially compresses the felt pad 296, shown in Figure 14, against the skin as mesh base 112 of fixture 110, shown in Figure 15, engages the cast 288, thereby approximating the pressure of the removed portion of the cast where the felt pad engages the skin.

In some embodiments, a portion of the mesh base 112 includes an adhesive. The adhesive may be used to temporarily affix the mesh base 112 to the cast until resin impregnates the mesh and affixes the mesh base 112 in a more permanent manner.

Referring to Figure17, a cap 308 for the fixture 110 is shown. The cap 308 is provided to maintain pressure on the body tissue exposed in fixture 110 when the ultrasonic treatment is completed. The cap 308 has a cylindrical portion 310 that extends into the aperture 134 of the fixture 110. The cap 308 has slotted lugs 312 on the cylindrical portion 310 that engage the bayonet lugs 306 in the fixture 110 The cylindrical felt pad 298 is positioned in the aperture 134 and the cylindrical portion 310 is inserted into the aperture 134 with the slotted lugs 312 offset from the bayonet lugs 306. The cap 308 is pressed against the cylindrical felt pad 298 until the pressure exerted by the cap 308 and cylindrical felt pad 298 against the skin approximates the pressure exerted by the cast 288 against the skin. The cylindrical felt pad 298 may also be comprised of substantially planar circular layers that may be removed one layer at a time in order to adjust the thickness of the felt pad and the resulting pressure against the skin. This pressure helps to inhibit window edemas. The cap 308 is then rotated so that its slotted lugs 312 engage the bayonet lugs 306. While the depicted embodiment includes the bayonet lugs 306 and slotted lugs 312, those skilled in the art would understand that other locking mechanisms may be used to connect the cap 308 to the support fixture 110.

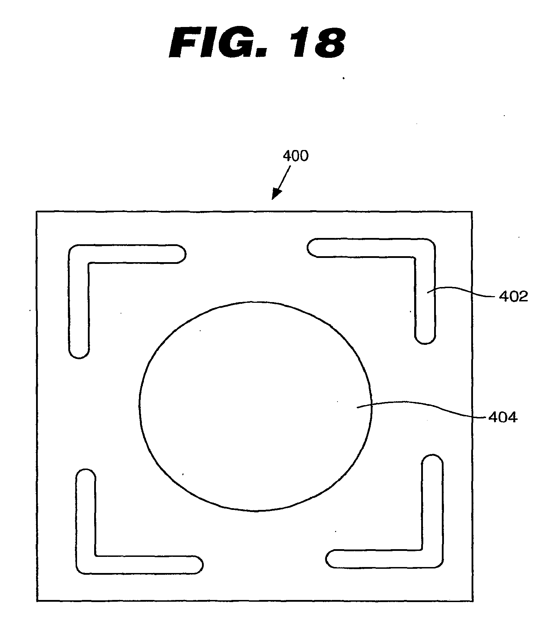

Figure 18 illustrates a second embodiment of the template for marking the opening 294, which is generally indicated by reference numeral 400. The template 400 is pressed against the cast 288 and centered on the mark 290 of the external location on the cast 288 of the bone fracture. The template 400 includes one or more slots 402 and a central cutout 404. In the depicted embodiment, the template 400 has four peripherally located slots 402. The cutout 404 is centered upon the mark 290, and the outline of the inner edges of the template opening is traced on the cast 288 via the slots 402. The traced portion of the cast is removed so that the opening 294 in the cast 288 exposes the skin, as shown in Figure 14.

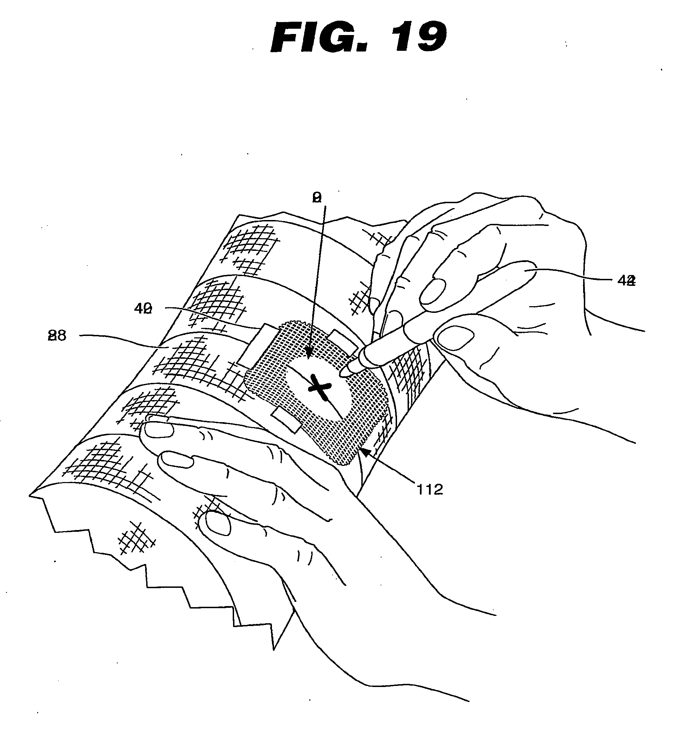

In some embodiments, the mesh base 112 may be used as a template to cut the opening 294. Figure 19 illustrates the mesh base 112 resting on the cast 288. The mesh base 112 is located relative to the mark 290. In Figure 19, the body 114 has been omitted for clarity and in order to reveal the mark 290. Optionally, adhesive tape 420 can be placed over the cast 288 and the mesh base 112 to temporarily hold the mesh base 112 while marking. Alternatively, an adhesive may be applied to a bottom surface of the mesh base 112 to temporarily affix it to the cast 288. After the mesh base 112 is located relative to the mark 290, a marking instrument 424, such as a pen or marker, is used to trace the outline of the mesh base 112. In some embodiments, the mesh base 112 has a varying weave spacing to allow the marking instrument 424 to be traced through the weave to allow reliable marking of the cast for cutting.

In yet other embodiments, the opening 294 may be cut free-hand. In other words, the opening 294 may be cut without a template. Once the opening 294 is cut, the mesh base 112 is trimmed with scissors or some other cutting device to match the shape of the mesh base 112 with the opening 294.

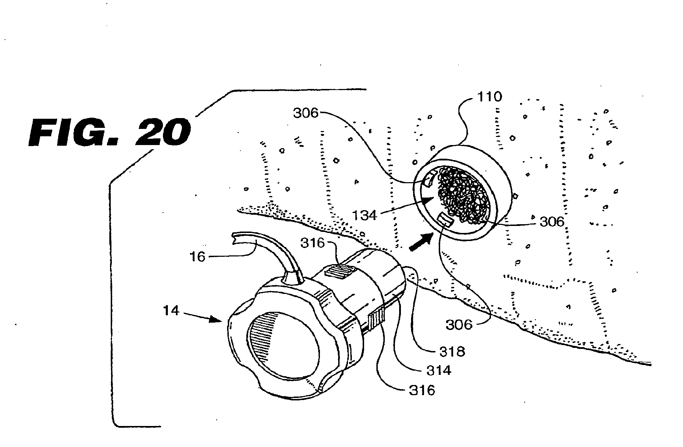

Figure 20 is a perspective view illustrating the alignment of the ultrasonic transducer treatment head module 14 with the fixture 110 for ultrasonic treatment of the injured bone. With the cap 308 and cylindrical felt pad 298, shown in Figure 17, removed, the projection 314 fits into the aperture 134 of the fixture 110 and the bore of the felt pad 296. The operative surface 318 of the transducer treatment head module 14 is pressed adjacent the skin. In some embodiments, the transducer treatment head module projection 314 has slotted lugs 316 that engage the bayonet lugs 306 in the fixture 110, and the transducer treatment head module 14 is rotated so that its slotted lugs 316 engage the bayonet lugs 306. The ultrasonic treatment then commences.

Referring again to Figures 3 and 4, to prevent inadvertent excitation of transducer treatment head module 14 and to insure compliance with treatment protocol, some embodiments include the fixture interlock 73, which includes switches on the outer surface of the transducer treatment head module. In this embodiment, slotted lugs 316 are fabricated from a conductive plastic and the bayonet lugs 306 in fixture 110 are electrically connected, such that when the slotted lugs 316 engage the bayonet lugs 306 an electrical path is completed between at least two of the slotted lugs 316. Suitable conductive plastics which may be utilized include conductive ABS plastics with either carbon, stainless steel, nickel or aluminum fibers.

The operative surface 318 of transducer treatment head module 14 includes a gel sensing element for confirming the presence of ultrasonic conductive material on the operative surface 318. This surface 318 is pre-coated with a coupling gel before it is inserted in the fixture 110 and engages the skin. Alternatively, the gel may be contained adjacent the operative surface 318 of transducer treatment head module 14 using a gel sack, gel bladder or like container.

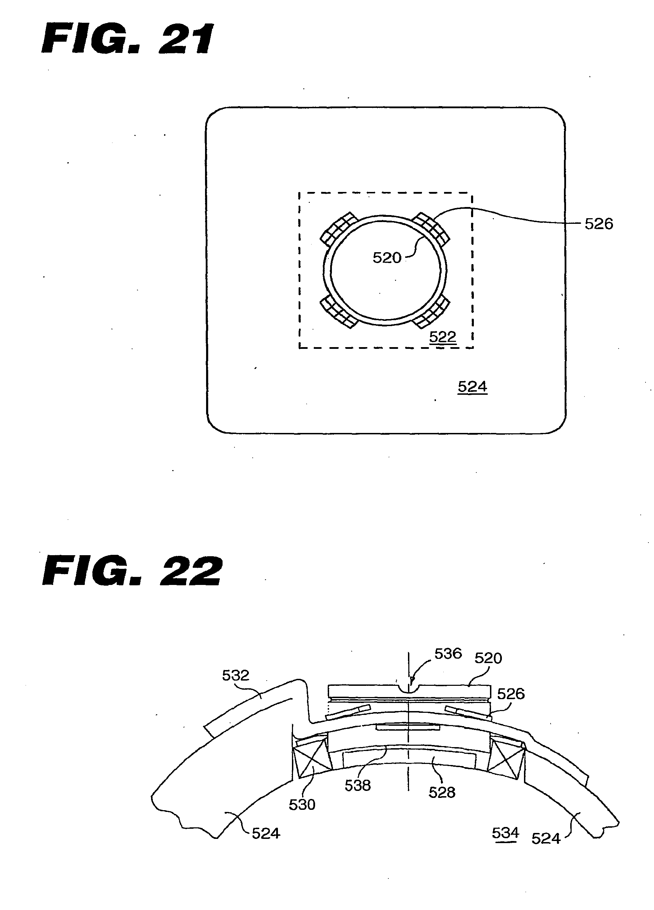

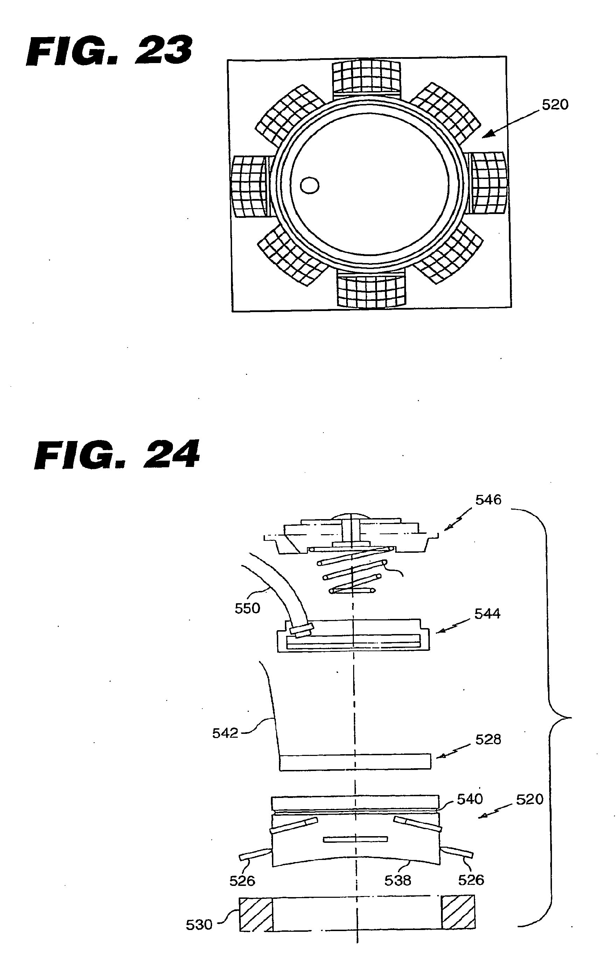

Figure 21 illustrates a further embodiment of the support fixture 520, positioned within a void 522 formed in a portion of a cast 524. Void 522 has a substantially square shape and is delineated by the dashed lines. Fixture 520 is shown having a substantially circular periphery and a plurality of mesh tabs 526 extending radially therefrom, as an alternative to the planar mesh base. Four mesh tabs 526 are visible in Figure 21. Additional mesh tabs 526 are hidden by cast material in a plane beneath the visible tabs as will become apparent in Figure 23. Fixture 520 preferably includes an axial bore within the substantially circular periphery to mount an ultrasound transducer to initiate a treatment, as will be discussed in further detail below. Fixture 520 is formed of a polymer, such as polypropylene or polyvinyl chloride.

Figure 22 illustrates a side cross-sectional view of fixture 520 within cast 524. Prior to placing fixture 520 into void 522, a spacer 530 is placed within void 522. Spacer 530 is configured to have a shape on its periphery which corresponds with the shape of void 522, and a hole in its center which corresponds to the shape of fixture 520. Spacer 530 is preferably formed of a medical grade felt or a similar material which will exhibit comfortable characteristics against a body portion of a patient, and may be fabricated in a plurality of layers so that the thickness can be adjusted depending on the thickness of cast 524.

Spacer 530 maintains fixture 520 at a predetermined distance from the body portion 534 of a patient, to prevent window edema or a similar injury to the patient due to uneven pressure at a casted site. As shown, fixture 520 is partially inserted into the hole within spacer 530 and is supported thereon by at least one of the radially extending mesh tabs 526. Mesh tabs 526 contain living hinges formed by a reduction in cross-section of the mesh tabs 526 at a proximal end adjacent the bore of fixture 520 which weakens mesh tabs 526 at the hinge point, thus allowing them to bend freely. The living hinges provide for lateral flexure of mesh tabs 526 to enhance the ability to conform to varying angles which are a function of the anatomy of the patient. Moreover, the living hinges allow fixture 520 to be articulated to correct for other angular misalignments.

Fixture 520 is secured within void 522 in cast 524 by weaving strips 532 of cast material between mesh tabs 526. A plurality of layers of cast material strips 532 are placed around fixture 520 until a desired thickness is achieved. Further, due to its mesh nature, the cast material impregnates the openings of the mesh tabs 526, thereby increasing the structural attachment of the fixture 520 to the cast 524. The configuration of fixture 520 having mesh tabs 526 allows the fixture to be installed before or after the cast is installed. Advantageously, when the layers of cast material strips cure, fixture 520 will be an integral part of the cast. Thus, any impact on the skin of the patient, which would otherwise be transferred through fixture 520, will be minimized as it is absorbed by the cast.

Fixture 520 may optionally include a hemispherical notch 536 in an upper end thereof to accommodate a cord extending from an ultrasound treatment head module while the module is positioned within the fixture. A lower end 538 of fixture 520 is preferably concave to correspond to fit a convex body portion 534 of a patient, without impacting the skin which may cause edema or a similar injury.

An ultrasound transmission-enhancing medium 528 is preferably positioned within spacer 530 adjacent a treatment location to minimize or eliminate an air gap between an ultrasound transducer head module and a treatment location. The ultrasound transmission-enhancing medium 528 is preferably a conductive gel bladder but may be simply gel.

The apparatus of the present invention is configured to adapt to and fit within a substantially rectangular or square-shaped void in a cast as shown in a top view thereof in Figure 23. Advantageously, the fixture 520 converts the void into a circular receptacle for receiving a corresponding circular-shaped ultrasound transducer head module.

Turning now to the exploded side view in Figure 24, and proceeding from the bottom, spacer 530 is shown in cross-section, having a hole therein configured to insertably receive fixture 520. Fixture 520 includes a plurality of mesh tabs 526 and a concave lower end 538. Fixture 520 preferably includes at least one circumferential groove 540 in an upper portion thereof. The purpose of the circumferential groove 540 is to enable the removal of at least one layer of fixture 520 to adjust the height of fixture 520 to correspond to a thickness of a cast. In the depicted embodiment of the ultrasound transmission-enhancing medium 528, a means for facilitating removal of the medium from fixture 520 is provided. In this embodiment, the means for facilitating removal is a lead 542 shown extending from medium 528.

An ultrasound transducer head module 544 is positioned adjacent ultrasound transmission-enhancing medium 528 within fixture 520. Cord 550 connects module 544 with electronic driving circuitry. Housing 546 is then inserted in the upper portion of fixture 520 to enclose the components within fixture 520. A bias element 548 extends from a bottom portion of housing 546. Bias element 548 may be a spring and, more particularly, may be a conical helical spring. The conical helical spring is advantageously configured to fully collapse within itself and will therefore require less space within fixture 520. A conical helical spring will also maintain a uniform force on ultrasound transducer head module 544 and will allow module 544 to pivot to conform to the shape of transmission-enhancing medium 528.

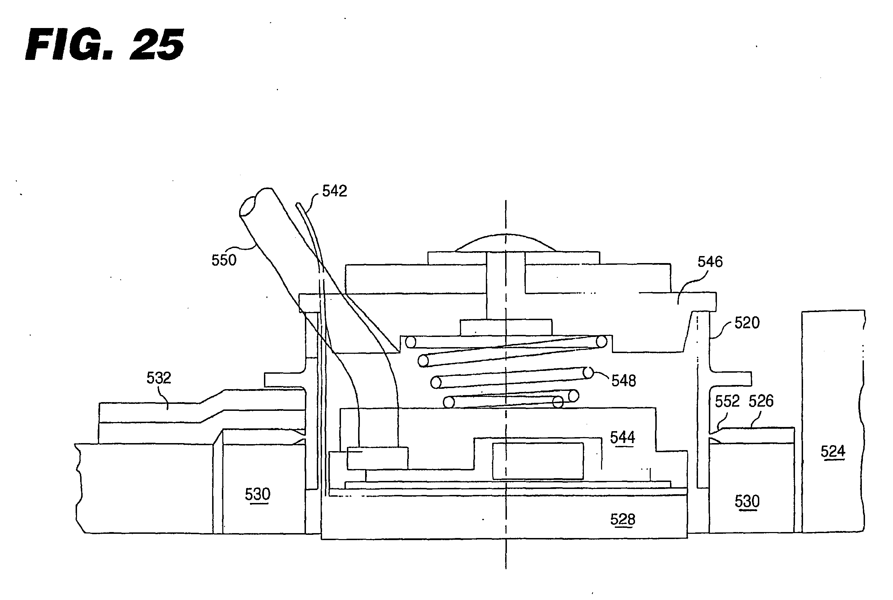

Figure 25 illustrates an enlarged side view of an assembled apparatus for mounting an ultrasound transducer in accordance with the present invention. This enlarged view illustrates the living hinge 552 on mesh tab 526 which allows for free lateral movement. Also shown, spring 548 in its compressed state urgingly biases transducer module 544 toward ultrasound transmission-enhancing medium 528.

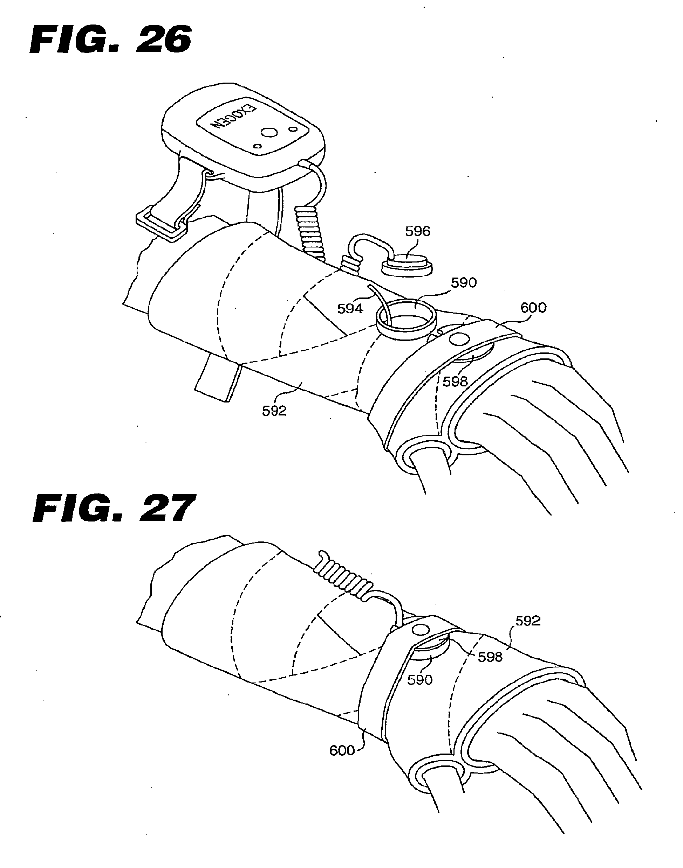

Referring to Figures 26 and 27, fixture 590 is shown secured within a cast 592 of a patient requiring ultrasound treatment. Lead 594 which is attached at its lower end to a transmission-enhancing medium is shown extending from fixture 590. Following the placement of ultrasound transducer head module 596 into fixture 590, cover 598 is placed over the top of fixture 590 and strap 600 is adjusted to secure the entire apparatus in place.

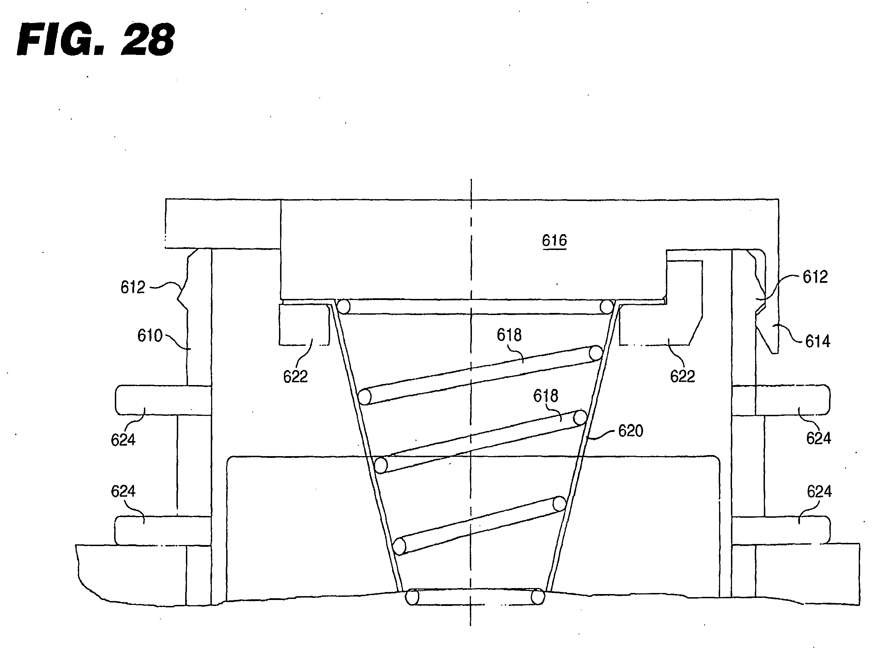

Figure 28 illustrates an embodiment of an apparatus for mounting an ultrasound transducer which features locking structure on the outside periphery of fixture 110 for retaining a transducer head module within the fixture. As illustrated, the locking structure includes a circumferential ridge 612 on the outside periphery of fixture 610 which is configured to engage at least one latch 614 extending downward from an outer periphery of cover 616. Although only one latch 614 is visible in Figure 28, it is preferable to have three latches extending from cover 616 and spaced about one hundred twenty degrees apart. Latch 614 is formed of a resilient material such that it will flex outward as the ridge thereon is forced over ridge 612, and it will snap back into position after it moves beyond ridge 612. The locking structure advantageously eliminates the need for a strap to secure the cover in place, as described above with other embodiments of the presently disclosed apparatus.

Conical helical spring 618 is held in contact with a lower surface of cover 616 by resilient housing 620. Resilient housing 620 is designed to maintain spring 618 in its position under cover 616 while exhibiting resiliency corresponding to the compressive property of spring 618. Housing 620 is secured to cover 616 by lock ring 622 which may be affixed to cover 616 by epoxy or any other means known to one having ordinary skill in the art. Housing 620 is preferably formed of polyurethane having a thickness of about 0.254mm to 2.54mm (about 0.01 inches to about 0.10 pinches).

Also illustrated in Figure 28 are flanges 624. It is contemplated that flanges 624 may be a plurality of separate continuous circumferential flanges, a single circumferential flange having a spiral configuration around the periphery of fixture 610 or at least one interrupted flange. The flanges 624 form a groove therebetween which may be used to receive casting material, casting tape, or a strap.

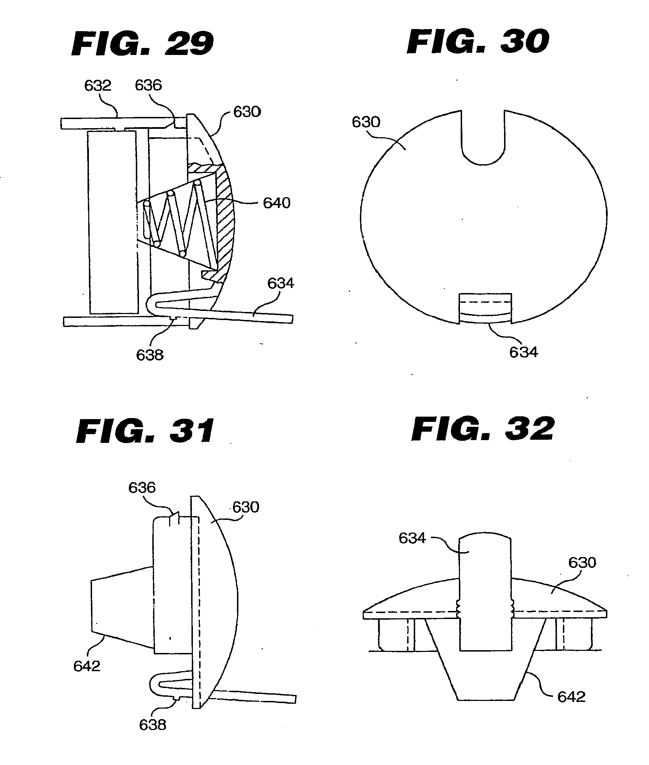

Figures 29-32 illustrate an alternative locking structure associated with cover 630 to removably engage cover 630 with fixture 632. In the cross-sectional view shown in Figure 29, cover 630 is illustrated locked within fixture 632 by means of a hinged locking tab 634 on a first side of cover 630 and a protrusion 636 on a second side of cover 630. To remove cover 630 from fixture 632, the portion of locking tab 634 which extends outwardly from cover 630 is depressed to release protrusion 638 from a groove formed on the inner surface of fixture 632. Cover 630 may then be pivoted upward to disengage protrusion 636 from a corresponding groove in fixture 632, and remove the cover. The disclosed locking structure advantageously eliminates the need for a strap to secure the cover in place, as described above with other embodiments of the presently disclosed apparatus. Furthermore, the configuration of locking tab 634 provides a structure for easily removing the cover by a single hand of the user. Alternatively, a cover may be provided with locking structure having two locking tabs. The cover may be removed by depressing one locking tab, similar to the embodiment described above, or by depressing both locking tabs simultaneously.

Similar to cover 616 illustrated in Figure 29, cover 630 includes a conical helical spring 640 which is held in contact with a lower surface of cover 630 by a resilient housing 642. Resilient housing 642 is designed to maintain spring 640 in its position under cover 630 while exhibiting resiliency corresponding to the compressive property of spring 640. Housing 642 is secured to cover 630 by a lock ring which may be affixed to cover 630 by epoxy or any other structure known to one having ordinary skill in the art.

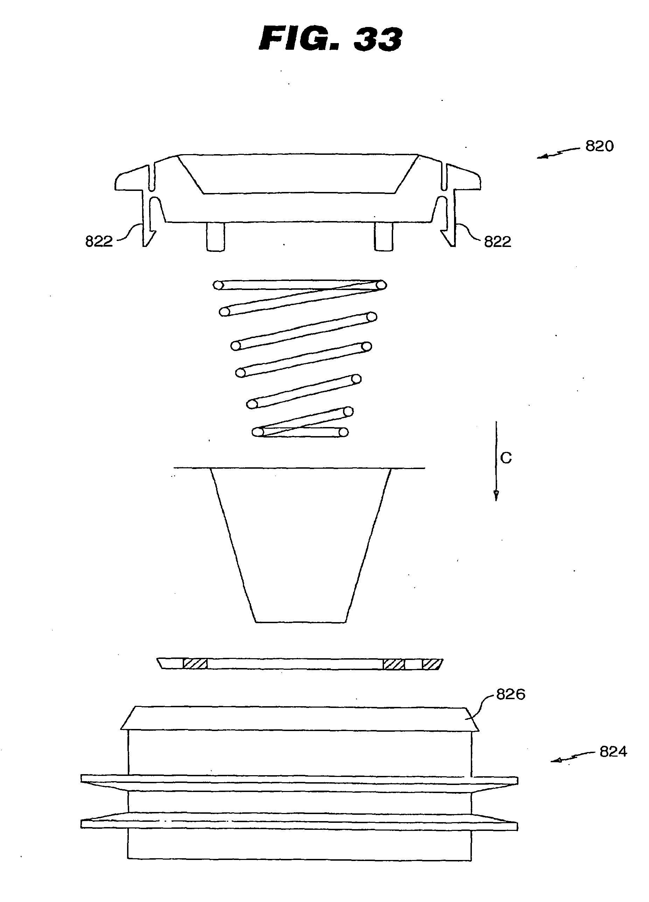

Additionally, as an alternative to the internal locking structure illustrated in Figures 29-32, Figure 33 illustrates an embodiment of the presently disclosed mounting apparatus which employs external locking structure. As shown in this exploded view, cover 820 includes external latches 822 integrally formed therewith. As cover 820 is moved in the direction of fixture 824, as indicated by Arrow C, the lower portions of latches 822 contact the circumferential lip 826 formed on the upper portion of fixture 824. As cover 820 continues in this direction, latches 822 are forced outwardly until the lower portions clear lip 826 and resiliently snap back to their original position, thereby locking cover 820 on fixture 824. Cover 820 may be removed from fixture 824 by depressing the upper portions of latches 822 in a direction toward the center of cover 820, and simultaneously lifting cover 820 off fixture 824.

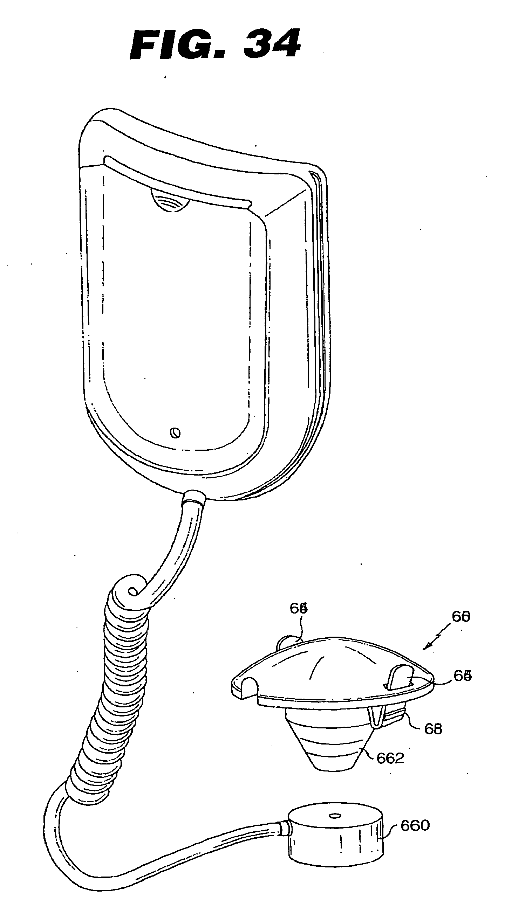

Figure 34 illustrates a perspective view of cover 650 having locking structure similar to that which was described above with reference to Figures 29-32. Cover 650 differs from cover 630 in that cover 650 has two locking tabs 654 for locking the cover within a fixture. Protrusion 658 is similarly formed on locking tab 654 to engage a groove on the inner surface of a fixture. Also shown in Figure 34 is an ultrasound treatment module with treatment head 660. Furthermore, conical helical spring 662 is connected to a lower surface of cover 650 to bias treatment head 660 in a direction toward a treatment site.

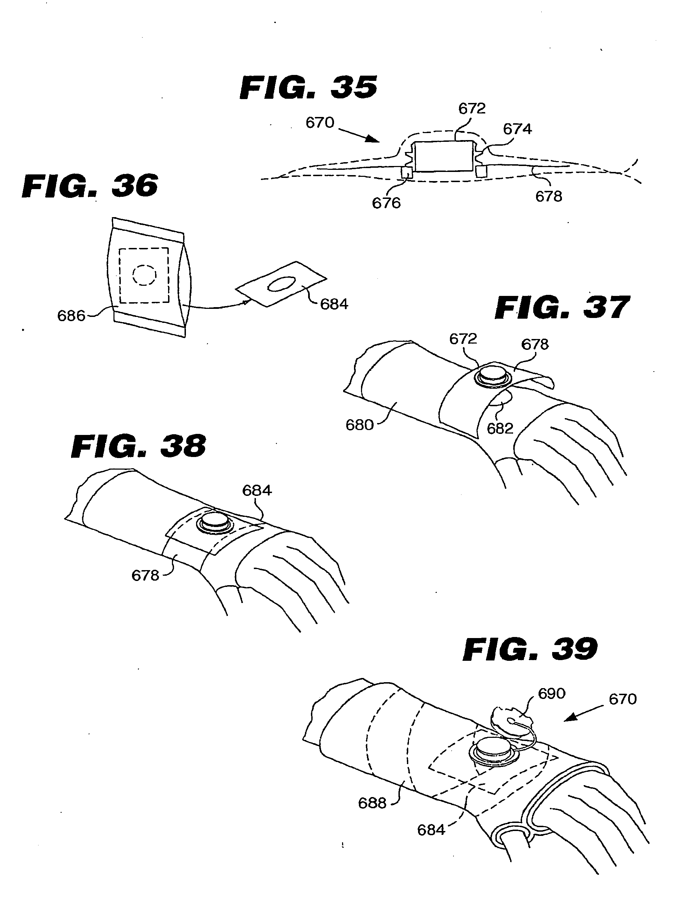

Figure 35 illustrates an apparatus 670 for the installation of an fixture adjacent a treatment location prior to installing a cast thereon for insertably receiving an ultrasound transducer treatment head. Apparatus 670 comprises a fixture 672 having radial flanges 674 on an outer periphery thereof, a spacer 676 to maintain fixture 672 a predetermined distance away from the skin of the patient, and padding portion 678 which wraps around the intended treatment location.

The pre-cast installation of apparatus 670 will now be described with reference to Figures 37-39. Referring initially to Figure 37, a stocking 680 is typically placed over the portion of the patient's body over which a cast will be installed. A hole 682 is then cut in stocking 680 at the precise location for receiving ultrasound treatment. Apparatus 670 is then positioned over stocking 680 such that fixture 672 is adjacent hole 682. Turning now to Figure 38, padding 678 is then draped around the intended treatment location and apparatus 670 is secured in place by a piece of casting tape 684. As illustrated in Figure 36, casting tape 684 is preferably supplied having a pre-cut hole therein and is stored in a sealed package 686 to maintain sterile conditions. Advantageously, the resin or adhesive of the casting tape 684 at least partially impregnates the mesh tabs of the fixture 672, thereby improving the mounting of the apparatus 670 to a cast 688. Casting tape 684 advantageously provides structural strength to apparatus 670 and simplifies the main cast wrapping. Referring now to Figure 38, apparatus 670 is shown secured within the main cast 688, ready for a cover 690 or an ultrasound transducer head module as discussed above.

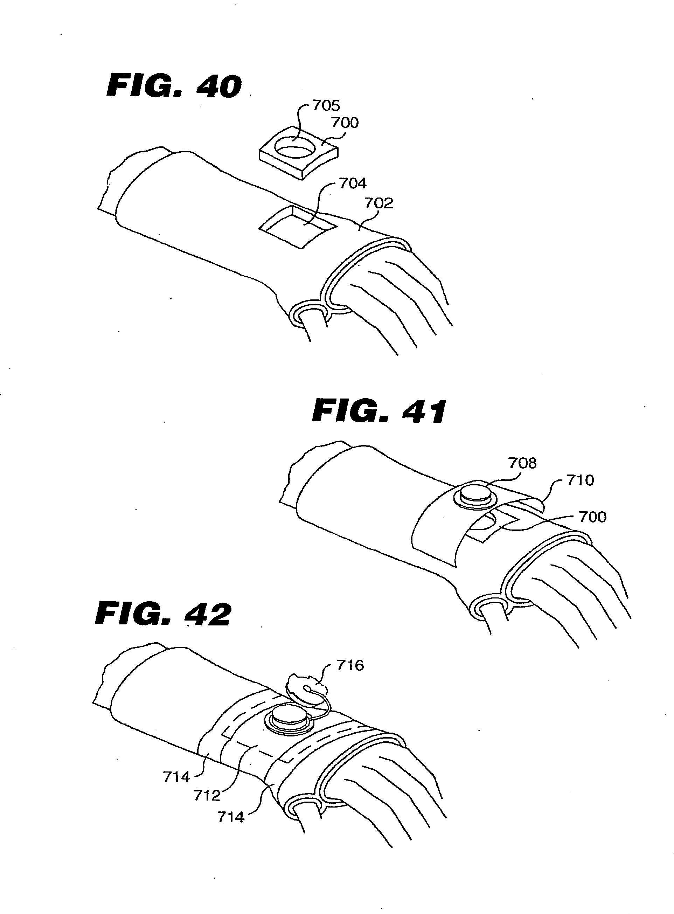

Turning now to Figure 40-42, a system for installing an apparatus for receiving an ultrasound treatment head module in a cast which has already been installed about a treatment location is illustrated. As shown in Figure 40, a felt pad 700 is provided to be placed within a void 704 cut in a cast 702. Felt pad 700, having a centrally located circular hole 705, is dimensioned corresponding to the thickness of cast 702. Advantageously, felt pad 700 may initially be used as a template for cutting void 704 in cast 702. Felt pad 700 is then installed within void 704. Referring to Figure 41, felt pad 700 is shown within void 704 adjacent a treatment location on a patient and apparatus 706 is positioned such that fixture 708 fits within the hole 705 in felt pad 700. Padding 710 is then draped around cast 702. Turning now to Figure 42, apparatus 706 is then secured in place with a precut piece of casting tape 712 which is configured and dimensioned to fit over fixture 708. Apparatus 706 and tape 712 may then be further secured in place by strips of about twenty-five millimeter wide casting tape 714.
Advantageously, the resin or adhesive of the casting tape 712 at least partially impregnates the mesh tabs of the fixture 708, thereby improving the mounting of the fixture 708 to the cast 702. A cover 716 or ultrasound transducer may then be placed in fixture 708.

The invention also includes a method of mounting a therapeutic device to an orthopaedic cast. The method includes the steps of: providing a support fixture, the support fixture comprising a body and a mesh projection extending from the body; placing a mark on the orthopaedic cast; making an opening in the orthopaedic cast relative to the mark; placing the support fixture over the opening; and placing cast material over the support fixture and the cast such that a portion of the cast material at least partially impregnates the mesh projection of the support fixture. Optionally, the support fixture further comprises an adhesive coating applied to the mesh base, and the adhesive coating is used to temporarily affix the mesh base to the cast until a portion of the cast material at least partially impregnates the mesh base. The method may further include the steps of locating a bone fracture and the step of placing a locating ring and strap on a cast. Alternatively, the method may include the step of placing a support fixture having peripheral markers on a cast. The method also may include the step of engaging a medical visualizing system. The method may further include the step of marking the opening relative to the mark using a template. Alternatively, the method may further include the step of marking the opening relative to the mark using the support fixture. The step of marking the opening relative to the mark using a template may include the step of outlining peripheral slots of the template. Alternatively, the step of marking the opening relative to the mark using the support fixture may include the step of placing adhesive tape over the support fixture and the cast.

In some embodiments, the invention may comprise a kit for ultrasonically treating injuries while maintaining patient mobility. The kit includes a support fixture configured and adapted to be worn by a patient adjacent an external site of an internal injury, the support fixture having a mesh base and a body extending from the mesh base; an ultrasonic transducer treatment head module including an ultrasonic signal generator and detachably engaged with the body of the support fixture; and a portable self contained main operating unit operatively connected to the treatment head module. The body of the support fixture may include an aperture configured to receive a portion of the ultrasonic transducer treatment head module. Further, the body may include one or more bayonet lugs extending into the aperture, which are electrically connected to form a conductive path therebetween. The ultrasonic transducer treatment head module may include one or more slotted lugs having at least a portion thereof extending from an outer surface of the transducer treatment head module and configured to engage the at least two bayonet lugs, the at least two slotted lugs being fabricated from conductive plastic such that when the slotted lugs engage the bayonet lugs a conductive path is formed between the slotted lugs. The ultrasonic signal generator may include signal generator circuitry and an internal power source connected to the signal generator circuitry, a display coupled to the signal generator circuitry to display treatment sequence data, a keypad coupled to the signal generator circuitry to permit user control and/or entry of data, the signal generator circuitry including a processor, a pulse RF signal generator, and a switch coupled to the processor for regulating the pulsed RF signal. Some embodiments of the kit may include a communication interface connected between a communication port and the processor to provide a communication link between the ultrasonic signal generator and an external computer. Additionally, some embodiments may include a cap engagable with the support fixture to replace the ultrasonic transducer treatment head module when the module is not being used for treatment. The may include a pad configured and adapted to be positioned between the cap and a skin surface of the patient for applying a predetermined pressure to the skin surface.

In view of the foregoing, it will be seen that the several advantages of the invention are achieved and attained.

The embodiments were chosen and described in order to best explain the principles of the invention and its practical application to thereby enable others skilled in the art to best utilize the invention in various embodiments and with various modifications as are suited to the particular use contemplated.

As various modifications could be made in the constructions and methods herein described and illustrated without departing from the scope of the invention, it is intended that all matter contained in the foregoing description or shown in the accompanying drawings shall be interpreted as illustrative rather than limiting. For example, while many illustrations depict the mesh base as having a rectangular shape, those of ordinary skill in the art would understand that the mesh base may have any number of shapes. Thus, the breadth and scope of the present invention should not be limited by any of the above-described exemplary embodiments, but should be defined only in accordance with the following claims appended hereto and their equivalents.

## Claims

1. An apparatus (10) for ultrasonically treating an injury in conjunction with an orthopaedic cast (288) the apparatus comprising:
a. a portable self-contained main operating unit (12);
b. a support fixture (110) configured and adapted for attachment to the orthopaedic cast adjacent an external site corresponding to an internal injury remote from said main operating unit, said support fixture having a body (114) and at least one mesh projection (112) extending from said body;
c. an ultrasonic transducer treatment head module (14) operatively connected to said main operating unit and detachably engaged with said body of said support fixture; and
d. casting material (684) for attaching the support fixture to the orthopaedic cast, wherein at least a portion of the casting material impregnates the at least one mesh projection.

2. The apparatus according to claim 1, wherein said at least one mesh projection comprises a planar mesh base.

3. The apparatus according to claim 1, wherein said at least one mesh projection comprises a plurality of mesh tabs (526).

4. The apparatus according to any of claims 1-3, wherein the at least one mesh projection further comprises at least one living hinge (552).

5. The apparatus according to any of claims 1-4, wherein the at least one mesh projection conforms to a small radius of curvature.

6. The apparatus according to any of claims 1-5, wherein said main operating unit has an internal power source (77) and is dimensioned to be carried by a patient during treatment.

7. The apparatus according to any of claims 1-6, wherein said treatment head module has an ultrasonic signal generator (124) and signal generator circuitry (38) operatively associated therewith.

8. The apparatus according to any of claims 1-7, wherein said treatment head module comprises an ultrasound transducer (85).

9. The apparatus according to claim 8, wherein said ultrasound transducer has piezoelectric properties and is made from a material selected from the group consisting of a ceramic material, a single-crystal relaxor ferroelectric, lead zirconate titanate, lead metaniobate, barium titanate, and piezoelectric co-polymers of polyvinylidene fluoride (PVDF).

10. The apparatus according to any of claims 1-9, wherein said treatment head module is connected to said main operating unit through a wireless connection.

11. The apparatus according to any of claims 1-6, wherein said main operating unit has an ultrasonic signal generator and signal generator circuitry, wherein said signal generator circuitry includes a processor, a pulsed signal generator, and a switch coupled to said processor for regulating said pulsed signal.

12. The apparatus according to claim 1, wherein a portion of the mesh projection is constructed of a material that may be seen with a chosen medical visualizing system.

13. The apparatus according to claim 12, wherein a portion of the mesh projection is at least partially opaque to X-radiation..

14. The apparatus according to claim 12, wherein a portion of the mesh projection is at least partially opaque to infra-red radiation.

15. The apparatus according to claim 12, wherein a portion of the mesh projection is at least partially paramagnetic.

16. The apparatus according to any of claims 1-15, wherein a portion of the mesh projection includes an adhesive coating.

17. The apparatus according to any of claims 1-16, wherein the mesh projection further comprises one or more peripheral markers (170).

## Patentansprüche

1. Ein Gerät (10) zur Behandlung einer Verletzung mit Ultraschall in Verbindung mit einem orthopädischen Gipsverband (288), wobei das Gerät Folgendes beinhaltet:
a. eine tragbare unabhängige Hauptbedieneinheit (12)
b. eine Stützhaltevorrichtung (110), die zur Befestigung an dem orthopädischen Gipsverband angrenzend an eine äußere Stelle, die einer inneren Verletzung entspricht, entfernt von der Hauptbedieneinheit konfiguriert und angepasst ist, wobei die Stützhaltevorrichtung einen Körper (114) und mindestens einen Netzvorsprung (112), der sich von dem Körper erstreckt, aufweist;
c. ein Kopfmodul (14) eines Wandlers zur Behandlung mit Ultraschall, das bedienbar mit der Hauptbedieneinheit verbunden ist und abnehmbar mit dem Körper der Stützhaltevorrichtung in Eingriff steht, und
d. einen Gusswerkstoff (684) zum Befestigen der Stützhaltevorrichtung an dem orthopädischen Gipsverband, wobei mindestens ein Abschnitt des Gusswerkstoffs den mindestens einen Netzvorsprung imprägniert.

2. Gerät gemäß Anspruch 1, wobei der mindestens eine Netzvorsprung einen ebenen Netzboden beinhaltet.

3. Gerät gemäß Anspruch 1, wobei der mindestens eine Netzvorsprung eine Vielzahl von Netzstreifen (526) beinhaltet.

4. Gerät gemäß einem der Ansprüche 1-3, wobei der mindestens eine Netzvorsprung ferner mindestens ein Kunststoffscharnier (552) beinhaltet.

5. Gerät gemäß einem der Ansprüche 1-4, wobei sich der mindestens eine Netzvorsprung an einen kleinen Krümmungsradius anpasst.

6. Gerät gemäß einem der Ansprüche 1-5, wobei die Hauptbedieneinheit eine interne Leistungsquelle (77) aufweist und bemessen ist, um während der Behandlung von einem Patienten getragen zu werden.

7. Gerät gemäß einem der Ansprüche 1-6, wobei das Kopfmodul zur Behandlung einen Ultraschallsignalgenerator (124) und einen Signalgeneratorschaltkreis (38), betriebsfähig damit verbunden, aufweist.

8. Gerät gemäß einem der Ansprüche 1-7, wobei das Kopfmodul zur Behandlung einen Ultraschallwandler (85) beinhaltet.

9. Gerät gemäß Anspruch 8, wobei der Ultraschallwandler piezoelektrische Eigenschaften aufweist und aus einem Material, das aus der Gruppe, die aus einem keramischen Material, einem Einkristall-Relaxorferroelektrikum, Blei-Zirkonat-Titanat, Bleimetaniobat, Bariumtitanat und piezoelektrischen Copolymeren von Polyvinylidenfluorid (PVDF) besteht, ausgewählt ist, gefertigt ist.

10. Gerät gemäß einem der Ansprüche 1-9, wobei das Kopfmodul zur Behandlung über eine drahtlose Verbindung mit der Hauptbedieneinheit verbunden ist.

11. Gerät gemäß einem der Ansprüche 1-6, wobei die Hauptbedieneinheit einen Ultraschallsignalgenerator und einen Signalgeneratorschaltkreis aufweist, wobei der Signalgeneratorschaltkreis einen Prozessor, einen Impulssignalgenerator und einen Schalter, der zum Regulieren des gepulsten Signals an den Prozessor gekoppelt ist, umfasst.

12. Gerät gemäß Anspruch 1, wobei ein Abschnitt des Netzvorsprungs aus einem Material konstruiert ist, das mit einem ausgesuchten medizinischen Visualisierungssystem gesehen werden kann.

13. Gerät gemäß Anspruch 12, wobei ein Abschnitt des Netzvorsprungs Röntgenstrahlung gegenüber wenigstens teilweise opak ist.

14. Gerät gemäß Anspruch 12, wobei ein Abschnitt des Netzvorsprungs Infrarotstrahlung gegenüber wenigstens teilweise opak ist.

15. Gerät gemäß Anspruch 12, wobei ein Abschnitt des Netzvorsprungs wenigstens teilweise paramagnetisch ist.

16. Gerät gemäß einem der Ansprüche 1-15, wobei ein Abschnitt des Netzvorsprungs eine haftende Beschichtung umfasst.

17. Gerät gemäß einem der Ansprüche 1-16, wobei der Netzvorsprung ferner eine oder mehrere periphere Markierungen (170) beinhaltet.

## Revendications

1. Un appareil (10) destiné à traiter une blessure par ultrasons en conjonction avec un plâtre orthopédique (288), l'appareil comprenant :
a. un boîtier de commande principal autonome portatif (12) ;
b. un dispositif de fixation formant support (110) configuré et adapté pour s'attacher sur le plâtre orthopédique adjacent à un site externe correspondant à une blessure interne, à distance dudit boîtier de commande principal, ledit dispositif de fixation formant support ayant un corps (114) et au moins une projection en tissu à mailles (112) qui s'étend à partir dudit corps ;
c. un module constituant tête de traitement à transducteur ultrasonore (14) connecté de façon opérationnelle audit boîtier de commande principal et en prise de façon à pouvoir être détaché avec ledit corps dudit dispositif de fixation formant support ; et
d. du matériau de plâtrage (684) destiné à attacher le dispositif de fixation formant support au plâtre orthopédique, dans lequel au moins une portion du matériau de plâtrage imprègne la au moins une projection en tissu à mailles.

2. L'appareil selon la revendication 1, dans lequel ladite au moins une projection en tissu à mailles comprend une base en tissu à mailles plane.

3. L'appareil selon la revendication 1, dans lequel ladite au moins une projection en tissu à mailles comprend une pluralité de pattes en tissu à mailles (526).

4. L'appareil selon n'importe lesquelles des revendications 1 à 3, dans lequel la au moins une projection en tissu à mailles comprend de plus au moins une charnière vive (552).

5. L'appareil selon n'importe lesquelles des revendications 1 à 4, dans lequel la au moins une projection en tissu à mailles se conforme à un petit rayon de courbure.

6. L'appareil selon n'importe lesquelles des revendications 1 à 5, dans lequel ledit boîtier de commande principal a une source d'énergie interne (77) et est dimensionné pour être porté par un patient durant le traitement.

7. L'appareil selon n'importe lesquelles des revendications 1 à 6, dans lequel ledit module constituant tête de traitement a un générateur de signal ultrasonore (124) et une circuiterie de générateur de signal (38) associée de façon opérationnelle à celui-ci.

8. L'appareil selon n'importe lesquelles des revendications 1 à 7, dans lequel ledit module constituant tête de traitement comprend un transducteur à ultrasons (85).

9. L'appareil selon la revendication 8, dans lequel ledit transducteur à ultrasons a des propriétés piézoélectriques et est réalisé à partir d'un matériau sélectionné dans le groupe consistant en un matériau céramique, un matériau ferroélectrique relaxeur monocristallin, du titanate-zirconate de plomb, du métaniobate de plomb, du titanate de baryum, et des copolymères piézoélectriques de polyfluorure de vinylidène (PVDF).

10. L'appareil selon n'importe lesquelles des revendications 1 à 9, dans lequel ledit module constituant tête de traitement est connecté audit boîtier de commande principal par une connexion sans fil.

11. L'appareil selon n'importe lesquelles des revendications 1 à 6, dans lequel ledit boîtier de commande principal a un générateur de signal ultrasonore et une circuiterie de générateur de signal, dans lequel ladite circuiterie de générateur de signal inclut un processeur, un générateur de signal pulsé, et un commutateur couplé audit processeur pour réguler ledit signal pulsé.

12. L'appareil selon la revendication 1, dans lequel une portion de la projection en tissu à mailles est construite en un matériau qui peut être vu avec un système de visualisation médical choisi.

13. L'appareil selon la revendication 12, dans lequel une portion de la projection en tissu à mailles est au moins en partie opaque aux rayons X.

14. L'appareil selon la revendication 12, dans lequel une portion de la projection en tissu à mailles est au moins en partie opaque aux rayons infrarouges.

15. L'appareil selon la revendication 12, dans lequel une portion de la projection en tissu à mailles est au moins en partie paramagnétique.

16. L'appareil selon n'importe lesquelles des revendications 1 à 15, dans lequel une portion de la projection en tissu à mailles inclut un revêtement adhésif.

17. L'appareil selon n'importe lesquelles des revendications 1 à 16, dans lequel la projection en tissu à mailles comprend de plus un ou plusieurs marqueurs périphériques (170).
